(19) Europäisches Patentamt
European Patent Office
Office européen des brevets

(11) Numéro de publication: **0 104 671**
**B1**

(12) **FASCICULE DE BREVET EUROPEEN**

(45) Date de publication du fascicule du brevet: **23.05.90**

(51) Int. Cl.[5]: **C 07 D 501/36,**
C 07 D 501/34, A 61 K 31/545

(21) Numéro de dépôt: **83200381.8**

(22) Date de dépôt: **13.02.81**

(60) Numéro de publication de la demande initiale en application de l' article 76 CBE: **0 034 536**

(54) **Nouvelles oximes dérivées de l'acide 3-alkyloxy ou 3-alkylthiométhyl-7 amino thiazolylacétamido céphalosporaniques, leur préparation, leur application comme médicaments et les compositions les renfermant.**

(30) Priorité: **18.02.80 FR 8003479**

(43) Date de publication de la demande:
**04.04.84 Bulletin 84/14**

(45) Mention de la délivrance du brevet:
**23.05.90 Bulletin 90/21**

(84) Etats contractants désignés:
**AT BE CH DE GB IT LI LU NL SE**

(56) Documents cités:
**EP-A-0 029 557**
**FR-A-2 119 074**
**FR-A-2 137 899**
**FR-A-2 348 218**
**FR-A-2 379 540**
**FR-A-2 385 722**

(73) Titulaire: **ROUSSEL-UCLAF**
**35, boulevard des Invalides**
**F-75007 Paris (FR)**

(72) Inventeur: **Heymes, René**
**93, rue Saint Martin**
**F-70000-Vesoul (FR)**
Inventeur: **Pronine, Didier**
**65, rue de la Ferone**
**F-93110-Rosny-sous-Bois (FR)**

(74) Mandataire: **Fritel, Hubert et al**
**Département des Brevets ROUSSEL UCLAF B.P. no 9**
**F-93230 Romainville (FR)**

**Le dossier contient des informations techniques présentées postérieurement au dépôt de la demande et ne figurant pas dans le présent fascicule.**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 104 671 B1

**Description**

La présente invention concerne de nouvelles oximes dérivées de l'acide 3-alkyloxy ou 3-alkylthiométhyl 7-amino thiazolyl acétamido céphalosporanique, leur procédé de préparation, leur application comme médicamments, les compositions les renfermant et les nouveaux intermédiaires obtenus.

La demande de brevet européen EP 0029.557 qui bénéficie d'une priorité antérieure à celle de la présente demande mais n'a été publiée qu'après la priorité de ladite présente demande décrit des produits proches des produits définis ci-après. Cependant, aucun de ces produits n'est décrit précisément dans le document cité.

La demande FR 2.385.722 décrit de façon générale des céphalosporines comportant un groupement amino thiazole et une fonction oxime sur la chaîne latérale. Un produit comparable aux produits de la présente demande est cité dans ce document. Cependant ce produit est pratiquement dérivé d'activité antibiotique.

Le brevet français 2.348.218 décrit des céphalosporines comportant un groupement amino thiazole et un radical méthoxyimino sur la chaîne latérale et en position 3 un groupement nucléophile attaché au noyau céphème par l'intermédiaire d'un groupement méthylène. En exemple figure le produit ayant un radical hydroxyméthyle en position 3.

Le brevet français 2.137.899 décrit de façon très générale des céphalosporines possédant sur la chaîne latérale un groupement hydroxyimino éthérifié. La Céfuroxime tombe dans cette formule générale.

L'invention a pour objet les produits de formule générale (I'):

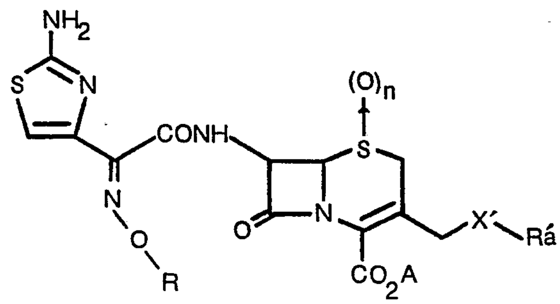

(I')

dans laquelle R, A, X', R'a, n ont les significations suivantes *ou bien:*

A) R représente,

*soit* un radical alkyle linéaire ou ramifié, alkényle, alkynyle ou cycloalkyle 40 ayant au plus 6 atomes de carbone,

*soit* un radical acétyle, propionyle, butyryle, valéryle, hexanoyle, acryloyle, crotonoyle, benzoyle, carbamoyle, chacun de ces radicaux étant éventuellement substitué par un ou plusieurs radicaux choisis parmi les radicaux carboxy éventuellement salifié ou estérifié, méthoxy carboxyle, éthoxy carbonyle, carbamoyle, diméthylcarbamoyle, amino, méthylamino, diméthylamino, halogène, méthoxy, éthoxy, propyloxy, méthylthio, éthylthio phényle, tétrazolyle, phénylthio, tétrazolylthio ou thiadiazolylthio éventuellement substitué par méthyle,

*soit* R représente un radical méthoxy carbonyle ou éthoxycarbonyle, A représente un atome d'hydrogène, un équivalent de métal alcalin, alcalinoterreux, de magnésium, d'ammonium ou d'une base organique aminée, ou A représente le reste d'un groupement ester facilement clivable, R'a représente un radical alkyle éventuellement interrompu par un hétéroatome, alkényle ou alkynyle ayant au plus 6 atomes de carbone linéaire ou ramifiée, ou un radical benzyle ou phénylethyle éventuellement substitué par un radical carboxy, amino ou diméthylaminoéthyle.

n est égal à 0, 1 ou 2,

X' représente un atome de soufre éventuellement oxydé sous forme de sulfoxyde ou de sulfone, ou un atome d'oxygène étant entendu que R ne représente pas un radical alkényle ou alkynyle ayant au plus 6 atomes de carbone ou un radical alkyle linéaire ou ramifié ayant au plus 6 atomes de carbone éventuellement substitué par ou plusieurs radicaux choisis parmi les radicaux carboxy éventuellement salifié ou estérifié, alcoxycarbonyle, amino, alkylamino, dialkylamino ou aryle hétérocyclique, lorsque le radical —X'—R'a représente un radical alkoxy, alkylthio ou alkénylthio.

*Ou bien* B] n est égal à 0, A représente un atome d'hydrogène et

*soit* R représente un radical $—CH_2—CH_2NH_2$, $—CH_2CO_2H$ ou $—C(CH_3)_2—CO_2H$, X' représente un atome d'oxygène ou de soufre et R' a représente un radical méthyle ou éthyle

*soit* R représente un radical

$$—\underset{\displaystyle CH_3}{\underset{|}{CH}}—CO_2H,$$

X' représente un atome d'oxygène ou de soufre et R'a représente un radical méthyle ainsi que les sels des

produits de formule (I') avec les acides minéraux ou organiques.

L'invention a notamment pour objet, parmi les produits de formule I', les produits répondant à la formule (I):

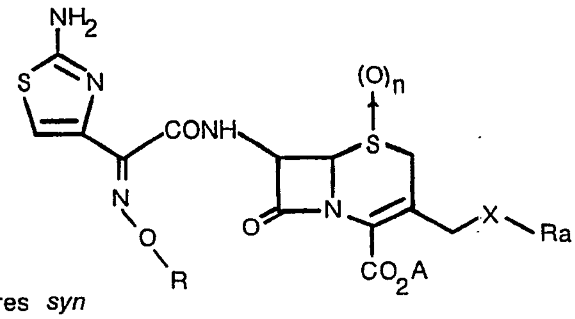

(I)

isomères *syn*

dans laquelle A, R et n sont définis comme précédemment, Ra représente un radical alkyle, alkényle ou alkynyle ayant au plus 6 atomes de carbone, linéaire ou ramifié, ou un radical benzyle ou phényléthyle éventuellement substitué par un radical carboxy, amino ou diméthylaminoéthyle et X représente un atome de soufre ou un atome d'oxygène, ainsi que les sels des produits de formule (I) avec les acides minéraux ou organiques.

Parmi les valeurs de R on peut citer:

a) les radicaux méthyle, éthyle, propyle, isopropyle, butyle, sec-butyle, tert-butyle, pentyle, iso-pentyle, sec-pentyle, tert-pentyle, néo-pentyle, hexyle, iso-hexyle, sec-hexyle, tert-hexyle;

b) les radicaux vinyle, allyle, 1-propényle, butényle, pentényle, hexényle;

c) les radicaux éthynyle, propargyle, butynyle;

d) les radicaux cyclopropyle, cyclobutyle, cyclopentyle, cyclohéxyle;

e) les radicaux acétyle, propionyle, butyryle, valéryle, hexanoyle, acryloyle, crotonoyle, benzoyle, carbamoyle, alkoxy carbonyle tel que méthoxy carbonyle ou éthoxycarbonyle.

Les radicaux indiqués ci-dessus aux paragraphes a) à d) peuvent eux-mêmes être substitués par un ou plusieurs radicaux tels que les radicaux:

carboxy éventuellement salifié ou esterifié, alkoxycarbonyle, tel que méthoxycarbonyle, ethoxycarbonyle; carbamoyle, di-méthylcarbamoyle; amino; di-alkylamino tel que diméthylamino, diéthylamino; alkylamino tel que méthylamino; halogène tel que chlore, brome, iode; alkoxy tel que methoxy, éthoxy, propyloxy; alkylthio tel que méthylthio, éthylthio; aryle tel que phényle; aryle hétérocyclique tel que tétrazolyle; arylthio tel que phénylthio éventuellement substitué, aryle hétérocyclique thio tel que tétrazolylthio, thiadiazolylthio éventuellement substitué par alkyle tel que méthyle.

Les radicaux cités au paragraphe d) peuvent de surcroit être substitués par un radical alkyle tel que défini au paragraphe a).

Parmi les valeurs de A on peut citer un équivalent de sodium, de potassium, de lithium, de calcium, de magnésium ou d'ammonium. On peut citer, parmi les bases organiques, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine.

On peut citer entre autres restes de groupements ester facilement clivables que peut représenter A, les groupements méthoxyméthyle, éthoxyméthyle, isopropyloxyméthyle, α-méthoxyéthyle, α-éthoxyéthyle, méthylthiométhyle, éthylthiométhyle, isopropylthiométhyle, pivaloyloxyméthyle, acétoxyméthyle, propionyloxyméthyle, butyryloxyméthyle, isobutyryloxyméthyle, valéryloxyméthyle, isovaléryloxyméthyle, tert-butyl carbonyloxyméthyle, hexadécanoyloxyméthyle, propionyloxyéthyle, isovaléryloxyéthyle, 1-acétyloxyéthyle, 1-propionyloxyéthyle, 1-butyryloxyéthyle, 1-tert-butylcarbonyloxyéthyle, 1-acétyloxypropyle, 1-hexadécanoyloxyéthyle, 1-propionyloxypropyle, 1-méthoxycarbonyloxyéthyle, méthoxycarbonyloxyméthyle, 1-acétyloxybutyle, 1-acétyloxyhexyle, 1-acétyloxyheptyle, phtalidyle, 5,6-diméthoxyphtalidyle, tert-butylcarbonylméthyl, allyle, 2-chloroallyle, méthoxycarbonylméthyle, benzyle ou tert-butyle.

On peut encore citer entre autres restes de groupements esters qui peut représenter A, les groupements méthoxyéthoxyméthyle, diméthylaminoéthyle, cyanométhyle, tert-butyloxycarbonylméthyle, 2,2-éthylènedioxyéthyle, cyanoéthyle, 2,2-diméthoxyéthyle, 2-chloroéthoxyméthyle, 2-hydroxyéthoxyéthyle, 2,3-époxypropyle, 3-diméthylamino, 2-hydroxypropyle, 2-hydroxyéthyle, 2-méthylaminoethoxyméthyle, 2-aminoéthoxyméthyle, 3-méthoxy, 2,4-thiadiazol-5-yle, 2-tétrahydropyranyle, 2-méthoxyprop-2-yle, 1-hydroxyprop-2-yle, isopropyle, carbamoyleméthyle, chlorométhyle, 2-chloroéthyle, acétyl méthyle, 2-méthylthioéthyle, ou thiocyanatométhyle.

On peut encore citer entre autres restes de groupements esters que peut représenter A, les groupements 2-chloro-1-acéthyoxyéthyle, 2-bromo 1-acétyloxyéthyle, 2-fluoro 1-acétyloxyéthyle, 2-méthoxy 1-acétyloxyéthyle, 2-méthyl 1-acétyloxypropyle, 2-acétyloxyprop-2-yle, 1-méthoxyacétyloxyéthyle, 1-acétylcarbonyloxyéthyle, 1-hydroxyacétyloxyéthyle, 1-formylcarbonyloxyéthyle, 1-(2-thiényl)carbonyloxyéthyle, 1-(2-furyl)carbonyloxyéthyle, 1-(5-nitro 2-furyl)carbonyloxyéthyle, 1-(2-pyrrolyl)carbonyloxyéthyle, 1-(propionyloxycarbonyloxyéthyle, 1-(propyloxycarbonyloxy)éthyle, 1-(isopropyloxycarbonyloxy)éthyle, 1-(méthoxyéthoxycarbonyloxy)éthyle, 1-(allyloxycarbonyloxy)éthyle, 1-(2,3-époxy)propyloxycarbonyloxy éthyle, 1-(2-furyl)méthoxycarbonyloxy éthyle, 1-(2-fluoro)éthyloxy-carbonyloxy éthyle, 1-(méthoxycarbonyloxy)propyle, (2-méthoxycarbonyloxy)prop-2-yle, (méthoxycarbonyloxy)chlorométhyle, 1-(méthoxycarbonyloxy)2-chloroéthyle, 1-(méthoxycarbonyloxy) 2-méthoxyéthyle, 1-(méthoxycarbonyloxy) 1-allyle.

R'a peut représenter l'un des radicaux cités ci-dessus pour le substituant R aux paragraphes a) à c), en particulier méthyle, éthyle, propyle, isopropyle, allyle. R'a peut également représenter un radical méthoxy méthyle, éthoxyméthyle.

Parmi les valeurs aralkyle que peut représenter R'a, on peut citer en particulier les radicaux benzyle et phényléthyle. Parmi les substituants éventuels des radicaux aralkyle on peut citer les radicaux carboxy, amino, aminoalkyle, alkyle, amino ou dialkylamino, dialkylaminoalkyle tel que diméthylaminoéthyle.

Les produits de formule (I') peuvent également se présenter sous forme de sels d'acides organiques ou de minéraux.

Parmi les acides avec lesquels on peut salifier le ou les groupements amino des produits (I'), on peut citer entre autres, les acides acétique, trifluoroacétique, maléïque, tartrique, méthanesulfonique, benzène sulfonique, p-toluène sulfonique, phosphorique, sulfurique, chlorhydrique, bromhydrique.

Les produits peuvent également se présenter sous forme de sels internes.

L'invention a plus particulèrement pour objet les produits de formule générale (I) telle que définie ci-dessus; dans laquelle R représente un atome d'hydrogène ou un radical alkyle linéaire ou ramifié ayant de 1 à 4 atomes de carbone, éventuellement substitué par un radical carboxylique libre, estérifié ou salifié, ou par un radical amino et Ra représente un radical alkyle ayant au plus 6 atomes de carbone et, parmi ceux-ci, les produits de formule (I) dans laquelle Ra représente un radical méthyle et n représente le nombre 0.

L'invention a également en particulier pour objet les produits de formule générale (I) telle que définie ci-dessus, répondant à la formule générale $I_A$.

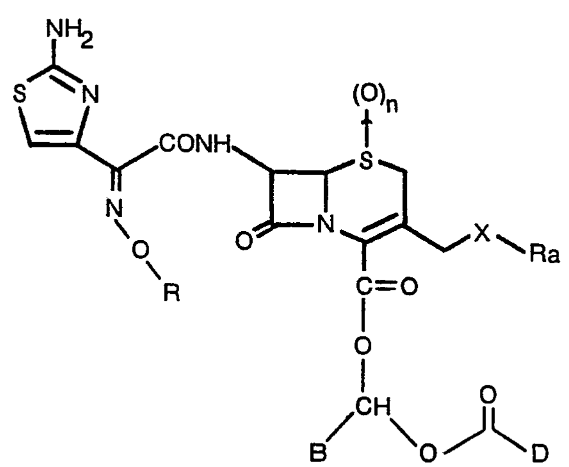

$(I_A)$

dans laquelle R, n, X et Ra sont définis comme précédemment, B représente un atome d'hydrogène ou un radical alkyle linéaire ou ramifié, renfermant de 1 à 5 atomes de carbone et D représente un radical alcoyle ou alcoxy linéaire ou ramifié, renfermant de 1 à 15 atomes de carbone et notamment de 1 à 5 atomes de carbone, ainsi que leurs sels avec les acides minéraux ou organiques.

Parmi les produits de formule $I_A$, on retient tout particulièrement ceux dans lesquels B représente un atome d'hydrogène ou un radical méthyle ou éthyle et D représente un radical méthyle, éthyle, méthoxy ou éthoxy.

L'invention a plus particulièrement pour objet les produits définis ci-après dans les exemples et spécialement

les acides 3-méthylthiométhyle 7-[[2-(2-amino thiazol-4-yl) 2-(2-bromoethyl)oximino]acétamido]ceph-3-ème 4-carboxylique, isomère syn, et 7-[[2-(aminothiazol-4-yl)2[(carboxy méthoxy imino]acétyl]amino 3-(méthylthiométhyl) ceph-3-ème 4-carboxylique isomère syn, ainsi que leurs sels avec les métaux alcalins, alcalino-terreux, le magnésium, l'ammoniaque, les bases organiques aminées, les acides et leurs esters facilement clivables.

Il est entendu que les produits de formule (I') précités peuvant exister,

*soit* sous la forme indiquée par ladite formule (I')
*soit* sous la forme de produits de formule ($I'_Z$):

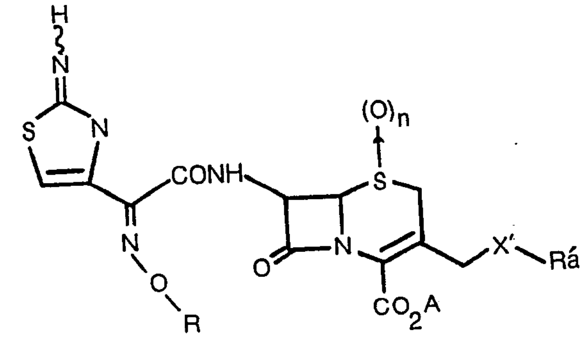

($I'_Z$)

dans laquelle R, R'a, A, X' et n ont la signification précédente.

L'invention concerne également un procédé de préparation des produits de formule (I') telle que définie ci-dessus caractérisé en ce que:

A) soit l'on traite un produit de formule (II)

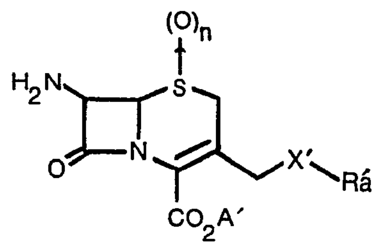

(II)

dans laquelle n, X' et R'a ont la signification précédente et A' représente un atome d'hydrogène ou le reste d'un groupement ester facilement éliminable, par un produit de formule (III)

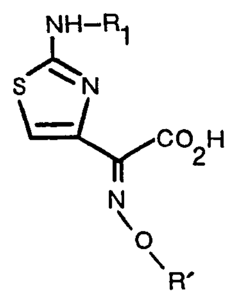

(III)

ou un dérivé fonctionnel de cet acide, formule (III) dans laquelle $R_1$ représente un atome d'hydrogène ou un groupement protecteur du radical amino et soit R' représente un atome d'hydrogène, un groupement protecteur du radical hydroxyle, un radical alkyle linéaire ou ramifié, alkényle, alkynyle ou cycloalkyle ayant au plus 6 atomes de carbone, soit R' représente un radical acyle, ou alcoxycarbonyle, chacun de ces radicaux étant éventuellement substitué, pour obtenir un produit de formule (IV):

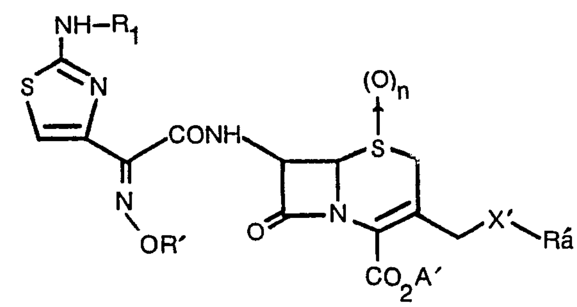

(IV)

dans laquelle $R_1$, R', A', R'a, X' et n ont la signification précédente,

B) soit l'on traite un produit de formule (V):

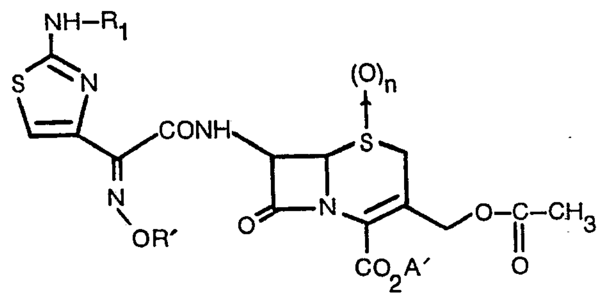

(V)

dans laquelle $R_1$, R', A' et n ont la signification précédente, *ou bien* par un produit de formule R'a—SH dans laquelle R'a a la signification précédente, *ou bien* d'abord par le 2-mercapto pyridine N-oxyde puis par un produit de formule R'aOH dans laquelle R'a a la signification précédente, pour obtenir un produit de formule (IV) tel que défini précedemment, produit de formule (IV) que, si désiré, dans le cas où n est égal à 0 et X' représente un atome de soufre ou d'oxygène, l'on traite par un agent d'oxydation pour obtenir un produit de formule (IV) dans laquelle n est égal à 1 ou 2 et X' représente un atome de soufre oxydé sous forme de sulfoxyde ou de sulfone, ou un atome d'oxygène et, produit de formule IV que l'on soumet si nécessaire ou si désiré, à l'une ou plusieurs des réactions suivantes, dans un ordre quelconque,

a) coupure par hydrolyse, hydrogénolyse ou par action de la thiourée de tout ou partie des groupements esters ou des groupements de protection du ou des radicaux amino ou du radical hydroxyle,

b) estérification ou salification par une base du ou des radicaux carboxyliques,

c) salification par un acide du ou des radicaux amino.

En plus des groupements cités ci-dessus, le groupement ester facilement éliminable que peut représenter A' peut être par exemple l'ester formé avec les radicaux butyle, isobutyle, tert-butyle, pentyle, hexyle, acétoxyméthyle, propionyloxyméthyle, butyryloxyméthyle, valéryloxyméthyle, pivaloyloxyméthyle, 2-acétoxyéthyle, 2-propionyloxyéthyle, 2-butyryloxyéthyle.

On peut également citer les radicaux 2-iodoéthyle, βββ-trichloroéthyle, vinyle, allyle, éthynyle, propynyle, benzyle, 4-méthoxybenzyle, 4-nitrobenzyle, phényléthyle, trityle, diphénylméthyle, 3,4-diméthoxyphényle.

On peut également citer les radicaux phényle, 4-chlorophényle, tolyle, tert-butylphényle.

Le groupement protecteur du radical amino que peut représenter $R_1$ peut être par exemple un radical alkyle de 1 à 6 atomes de carbone tel que, préférentiellement, tert-butyle ou tert-amyle, $R_1$ peut également représenter un groupement acyle, aliphatique, aromatique ou hétérocyclique ou un groupe carbamoyle.

On peut citer les groupements alcanoyles inférieurs tel que par exemple formyle, acétyle, propionyle, butyryle, isobutyryle, valéryle, isovaléryle, oxalyle, succinyle, pivaloyle. $R_1$ peut également représenter un groupe alkoxy, ou cycloalkoxycarbonyle inférieur tel que par exemple, méthoxycarbonyle, éthoxycarbonyle, propoxycarbonyle, 1-cyclopropyléthoxycarbonyle, isopropyloxycarbonyle, butyloxycarbonyle, tert-butyloxycarbonyle, pentyloxycarbonyle, hexyloxycarbonyle, un groupe benzoyle, toluolyle, naphtoyle, phtaloyle, mésyle, phénylacétyle, phénylpropionyle, un groupe aralkoxycarbonyle, tel que benzyloxycarbonyle.

Les groupements acyle peuvent être substitués par exemple par un atome de chlore, de brome, d'iode ou de fluor. On peut citer les radicaux chloroacétyle, dichloroacétyle, trichloroacétyle, bromoacétyle ou trifluoroacétyle.

$R_1$ peut également représenter un groupement aralkyle inférieur tel que benzyle, 4-méthoxybenzyle ou phényléthyle, trityle, 3,4-di-méthoxybenzyle ou benzhydryle.

$R_1$ peut également représenter un groupe haloalkyle tel que trichloroéthyle.

$R_1$ peut également représenter un groupement chlorobenzoyle, para-nitrobenzoyle, para-tert-butylbenzoyle, phénoxyacétyle, caprylyle, n-décanoyle, acryloyle, trichloroéthoxycarbonyle.

$R_1$ peut également représenter un groupement méthylcarbamoyle, phénylcarbamoyle, naphtylcarbamoyle, ainsi que les thiocarbamoyles correspondants.

La liste ci-dessus n'est par limitative, il est évident que d'autres groupements protecteurs des amines, groupements connus en particulier dans la chimie des peptides, peuvent également être utilisés.

Le groupement de protection du radical hydroxyle que peut représenter R', peut être choisi dans la liste ci-dessous: R' peut représenter un groupe acyle tel que par exemple formyle, acétyle, chloroacétyle, bromoacétyle, dichloroacétyle, trichloroacétyle, trifluoroacétyle, méthoxyacétyle, phénoxyacétyle, benzoyle, benzoylformyle, p-nitrobenzoyle. On peut citer également les groupements éthoxycarbonyle, méthoxycarbonyle, propoxycarbonyle, βββ-trichloroéthoxycarbonyle, benzyloxycarbonyle, tert-butoxycarbonyle, 1-cyclo propyléthoxycarbonyle, tétrahydropyrannyle, tétrahydrothiopyrannyle, méthoxytétrahydropyrannyle, trityle, benzyle, 4-méthoxybenzyle, benzhydryle, trichloroéthyle, 1-méthyl 1-méthoxyéthyle, phtaloyle.

On peut également citer d'autres acyles tels que propionyle, butyryle, isobutyryle, valéryle, isovaléryle, oxalyle, succinyle et pivaloyle.

On peut également citer els radicaux phénylacétyle, phénylpropionyle, mésyle, chlorobenzoyle, para-nitrobenzoyle, par-tert-butylbenzoyle, caprylyle, acryloyle, méthylcarbamoyle, phénylcarbamoyle, naphtylcarbamoyle.

Dans un mode préférentiel d'éxécution du procédé, on traite le produit de formule (II) par un dérivé fonctionnel d'un produit de formule (III). Ce dérivé fonctionnel peut être par exemple un halogénure, un anhydride symétrique ou mixte, l'amide, l'azide, ou un ester activé.

Comme exemple d'anhydride mixte on peut citer par exemple celui formé avec le chloroformiate d'isobutyle et celui formé avec le chlorure de pivaloyle et les anhydrides mixtes carboxylique-sulfonique formé par exemple avec le chlorure de para-toluène sulfonyle.

Comme exemple d'ester activé, on peut mentionner l'ester formé avec le 2,4-dinitrophénol et celui formé avec l'hydroxybenzothiazole.

Comme exemple d'halogénure, on peut citer le chlorure ou le bromure.

L'anhydride peut être formé in situ par action de carbodiimide NN'di substitué, par exemple la N,N-dicyclohexylcarbodiimide.

La réaction d'acylation est conduite de préférence dans un solvant organique tel que le chlorure de méthylène. On peut cependant utiliser d'autres solvants tels que le tétrahydrofuranne, le chloroforme ou le diméthylformamide.

Lorsqu'on utilise un halogénure d'acide et de manière générale lorsqu'une molécule d'acide halohydrique est libérée au cours de la réaction, on réalise la réaction de préférence en présence d'une base telle que la soude, la potasse, les carbonates et carbonates acides de sodium ou de potassium, l'acétate de sodium, la triéthylamine, la pyridine, la morpholine ou la N-méthylmorpholine.

La température de réaction est en général inférieure ou égale à la température ambiante.

Lorsque $R_1$ représente un atome d'hydrogène on utilise de préférence un anhydride mixte carboxylique sulfonique.

L'action du produit de formule R'aSH sur le produit de formule (V) est réalisée de préférence dans les conditions décrites dans le brevet français 2 379 540. On opére alors en présence d'éthérate de trifluorure de bore dans l'acide acétique ou le nitrométhane.

L'action, sur le produit de formule (V) d'abord du 2-mercapto pyridine N-oxyde puis de l'alcool R'aOH est effectuée de préférence dans les conditions indiquées dans le brevet français 2 119 074.

La formation de l'éther est effectuée de préférence en présence de sels cuivriques tels que le chlorure cuivrique.

L'oxydation du produit de formule (IV) peut être effectuée par un peracide, par exemple l'acide peracétique, per-phthalique, m-chloroperbenzoïque, ou perbenzoïque, ou par l'eau oxygénée.

Selon les valeurs de $R_1$, R' et A', les produits de formule (IV) peuvent ou non constituer des produits de formule (I).

Les produits de formule (IV) constituent des produits de formule (I) lorsque $R_1$ représente un atome d'hydrogène, lorsque R' ne représente par un groupement protecteur du radical hydroxyle que l'on désire éliminer et lorsque A' ne représente pas, parmi les groupements esters facilement clivables; l'un de ceux que l'on désirerait éliminer.

Dans les autres cas, l'action sur le produit de formule (IV) d'un ou plusieurs agents d'hydrolyse, d'hydrogénolyse ou de la thiourée a pour but d'éliminer le radical $R_1$ lorsque celui-ci représente un radical protecteur du radical amino, d'éliminer le radical R' lorsque celui-ci est différent de R et/ou d'éliminer le radical A' lorsque celui-ci représente, parmi les groupements ester facilement clivables l'un de ceux que l'on désire éliminer.

Cependant il est bien entendu possible d'éliminer $R_1$ sans toucher aux substituants R' et A' lorsque ceux-ci doivent être conservés. Il en est ainsi par exemple lorsque A' représente un groupement ester que l'on souhaite conserver tel qu'un groupement propionyloxyméthyle.

La nature des réactifs à mettre en jeu dans un tel cas est bien connu de l'homme de métier. Des exemples de telles réactions sont données plus loin dans la partie expérimentale.

On donne ci-après une énumération non exhaustive des moyens pouvant être mis en oeuvre pour éliminer les différents groupements.

L'élimination du groupe R1 peut être effectuée par hydrolyse, celle-ci étant acide, basique ou utilisant l'hydrazine.

On utilise préférentiellement l'hydrolyse acide pour éliminer les groupements alkoxy et cycloalkoxycarbonyles éventuellement substitués, tels que tert-pentyloxycarbonyle ou tert-butyloxycarbonyle, les groupements aralcoxycarbonyles éventuellement substitués tels que benzyloxycarbonyle, les groupements trityle, benzhydryle, tert-butyle ou 4-méthoxybenzyle.

L'acide que l'on utilise de préférence peut être choisi dans le groupe constitué par les acides chlorhydrique, benzène sulfonique ou para-toluène sulfonique, formique ou trifluoroacétique. On peut cependant utiliser d'autres acides minéraux ou organiques.

L'hydrolyse basique est utilisée préférentiellement pour éliminer les groupements acyle tels que trifluoroacétyle.

La base que l'on utilise de préférence est une base minérale telle que l'hydroxyde de sodium ou de

potassium. On peut également utiliser la magnésie, la baryte ou un carbonate ou carbonate acide de métal alcalin tel que les carbonates et carbonates acides de sodium ou de potassium ou d'autres bases.

On peut également utiliser l'acétate de sodium ou de potassium.

L'hydrolyse utilisant l'hydrazine est utilisée de préférence pour éliminer des groupes tels que phtaloyle.

Le groupement $R_1$ peut également être éliminé par le système zinc-acide acétique (pour le groupement trichloroéthyle), les groupements benzhydryle, benzyloxycarbonyle sont éliminés de préférence par l'hydrogène en présence d'un catalyseur.

Le groupement chloroacétyle est éliminé par action de la thio-urée en milieu neutre ou acide selon le type de réaction décrit par MASAKI J.A.C.S., *90*, 4508, (1968).

On peut également utiliser d'autres méthodes de déprotection connues dans la littérature.

Parmi les groupes préférés, on peut citer les groupements formyle, acétyle, éthoxycarbonyle, mésyle, trifluoroacétyle, chloroacétyle, trityle.

L'acide que l'on utilise de préférence est l'acide trifluoroacétique.

L'élimination du radical A' ou R', lorsque celle-ci est nécessaire, est réalisée dans des conditions semblables à celles décrites précédemment pour l'élimination de $R_1$.

On peut utiliser, entre autres, l'hydrolyse acide pour éliminer les radicaux alkyle ou aralkyle éventuellement substitués.

On utilise préférentiellement un acide choisi dans le groupe formé par les acides chlorhydrique, formique, trifluoroacétique et para-toluène sulfonique.

Les autres valeurs des radicaux A' ou R' sont, lorsque cela est désiré, éliminées selon les procédés connus de l'homme de métier. On opère de préférence dans des conditions modérées, c'est-à-dire, à température ambiante ou en chauffant légèrement.

Naturellement, on peut, lorsque par exemple $R_1$ et A' ou R' sont des groupements éliminables appartenant à des types différents, faire agir sur les produits (IV) plusieurs agents envisagés dans les énumérations précédentes.

La salification des produits peut être effectuée selon les méthodes usuelles.

La salification peut, par exemple, être obtenue par action sur un produit sous forme acide ou sur un solvat, par exemple le solvat éthanolique ou un hydrate de cet acide, d'une base minérale telle que l'hydroxyde de sodium ou de potassium, le carbonate ou le carbonate acide de sodium ou de potassium. On peut également utiliser les sels d'acides minéraux tels que le phosphate tri-sodique. On peut également faire appel à des sels d'acides organiques.

Comme sels d'acides organiques, on peut mentionner, par exemple, les sels de sodium d'acides carboxyliques aliphatiques linéaires ou ramifiés, saturés ou insaturés de 1 à 18 et de préférence de 2 à 10 atomes de carbone. Les chaines aliphatiques de ces acides peuvent être interrompues par un ou plusieurs hétéroatomes tels que l'oxygène ou le soufre ou substituées par des radicaux aryle, comme par exemple; phényle, thiényle, furyle, par un ou plusieurs radicaux hydroxyle ou par un ou plusieurs atomes d'halogène tels que fluor, chlore ou brome, préférentiellement chlore, par un ou plusieurs radicaux carboxyliques ou alkoxycarbonyles inférieurs, de préférence méthoxycarbonyle, éthoxycarbonyle ou propyloxycarbonyle, par un ou plusieurs radicaux aryloxy, de préférence phénoxy.

De plus, on peut utiliser comme acides organiques, des acides aromatiques suffisamment solubles comme par exemple des acides benzoïques substitués, de préférence par des radicaux alkyles inférieurs.

Comme exemples de tels acides organiques on peut mentionner: les acides formique, acétique, acrylique, butyrique, adipique, isobutyrique, n-caproïque, isocaproïque, chloropropioniques, crotonique, phénylacétique, 2-thiénylacétique, 3-thiénylacétique, 4-éthylphénylacétique, glutarique, l'ester monoéthylique de l'acide adipique, les acides hexanoïque, heptanoïque, décanoïque, oléïque, stéarique, palmitique, 3-hydroxypropionique, 3-méthoxypropionique, 3-méthylthiobutyrique, 4-chlorobutyrique, 4-phénylbutyrique, 3-phénoxybutyrique, 4-éthylbenzoïque, 1-propylbenzoïque.

On utilise cependant de préférence comme sels de sodium l'acétate de sodium, le 2-éthyl héxanoate de sodium ou le diéthyl acétate de sodium.

La salification peut également être obtenue par action d'une base organique comme la triéthylamine, la diéthylamine, la triméthylamine, la propylamine, la N,N-diméthyléthanolamine, le tris (hydroxyméthyl) amino méthane, la méthylamine, l'éthanolamine, la pyridine, la picoline, la dicyclohexylamine, la morpholine et la benzylamine.

Elle peut également être obtenue par action de l'arginine, de la lysine, de la procaïne, de l'histidine, de la N-méthyl glucamine.

Cette salification est réalisée de préférence dans un solvant ou un mélange de solvants tels que l'eau, l'éther éthylique, le méthanol, l'éthanol ou l'acétone.

Les sels sont obtenus sous forme amorphe ou cristallisée selon les conditions réactionnelles employées.

Les sels cristallisés sont préparés de préférence en faisant réagir les acides libres avec l'un des sels des acides carboxyliques aliphatiques mentionnés ci-dessus, de préférence, avec l'acétate de sodium.

La salification des produits par les acides minéraux ou organiques est effectuée dans les conditions usuelles.

L'estérification éventuelle des produits est effectuée dans les conditions classiques. On opère en

général en faisant réagir l'acide de formule (I') ou un dérivé fonctionnel, avec un dérivé de formule:

Z—Re

dans laquelle Z représente un radical hydroxyle ou un atome d'halogène tel que le chlore, le brome, l'iode et Re désigne le groupement ester à introduire, groupement dont une liste non exhaustive figure ci-dessus. Dans certains cas il peut être avantageux d'effectuer une estérification sur un produit dont l'amine et/ou un éventuel groupement oxyimino sont bloqués avant d'enlever le groupement protecteur de l'amine et du groupement oxyimino.

La présente invention concerne, plus spécialement, un procédé, tel que décrit ci-dessus, pour la préparation des produits de formule (I') telle que décrite précédemment, caractérisé en ce que l'on utilise, pour la mise en oeuvre du procédé, un produit de formule (III) dans laquelle $R_1$ représente un groupement protecteur du radical amino et en ce que le dérivé fonctionnel de l'acide de formule (III) est un anydride mixte carboxylique sulfonique.

L'anhydride carboxylique sulfonique utilisé est de préférence formé avec l' acide para-toluène sulfonique.

Le groupement protecteur que représente $R_1$ est de préférence le groupement trityle.

La présente demande a également pour objet un procédé de préparation du produit de formule (I'), telle que définie ci-dessus, caractérisé en ce que l'on traite un produit de formule (II')

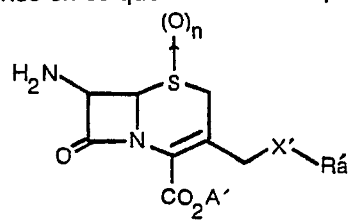

(II')

dans laquelle R'a, n, X et A' ont la signification précédente par un produit de formule (III')

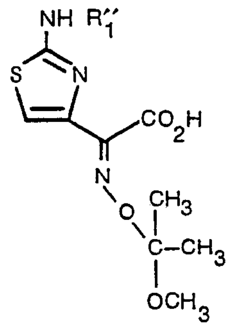

(III')

ou un dérivé fonctionnel de cet acide, formule (III') dans laquelle $R''_1$ représente un groupement protecteur du radical amino, pour obtenir un produit de formule (IV')

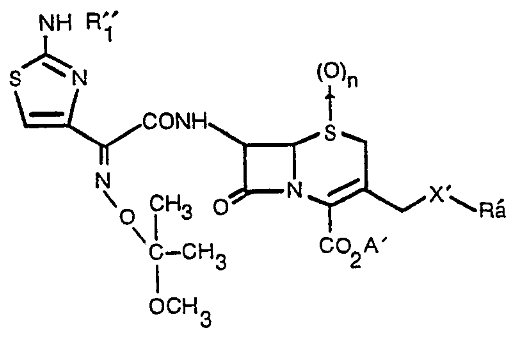

(IV')

dans laquelle A', $R''_1$, R'a, X' et n ont la signification précédente, produit de formule (IV') que l'on traite par un acide dans des conditions modérées, pour obtenir un produit de formule (VI)

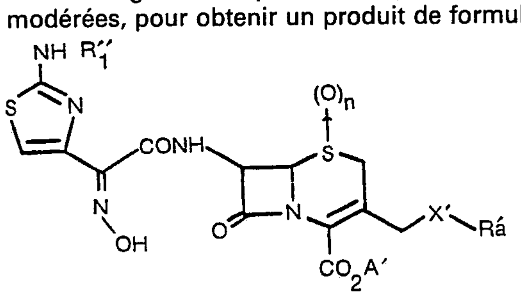

(VI)

produit que, si désiré, l'on estérifie ou salifie et traite en présence d'une base, par un dérivé fonctionnel de formule

Rd-Hal

dans laquelle Rd représente un radical alkyle linéaire ou ramifié, alkényle, alkynyle, ou cycloalkyle ayant au plus 6 atomes de carbone,

*soit* un radical acétyle, propionyle, butyryle, valéryle, hexanoyle, acryloyle, crotonoyle, benzoyle, carbamoyle, chacun de ces radicaux étant éventuellement substitué par un ou plusieurs radicaux choisis parmi les radicaux carboxy éventuellement salifié ou estérifié, alkoxycarbonyle, carbamoyle, diméthylcarbamoyle, amino, alkylamino, dialkylamino, halogène, alkoxy, alkylthio, aryle, aryle hétérocyclique, arylthio, aryle hétérocyclique thio éventuellement substitué par alkyle,

*soit* Rd représente un radical alkoxycarbonyle et Hal représente un atome d'halogène, pour obtenir un produit de formule VII

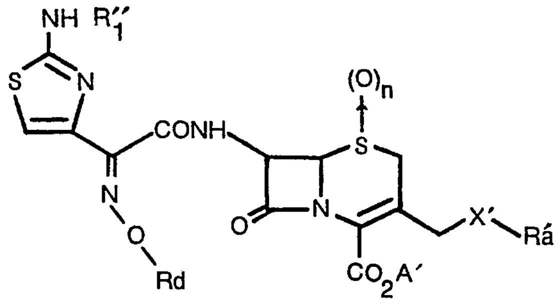

(VII)

dans laquelle $R''_1$, R'a, Rd, A', X' et n ont la signification indiquée ci-dessus et produits de formule VI et VII que l'on soumet à une hydrolyse, hydrogénolyse ou à l'action de la thiourée pour éliminer le radical $R''_1$, de protection du radical amino et, si désiré, ou si nécessaire, à l'une ou plusieurs des réactions suivantes, dans un ordre quelconque:

a) élimination du ou des groupements esters,

b) estérification ou salification par une base, du ou des radicaux carboxyliques,

c) salification par un acide du ou des radicaux amino.

Le groupement protecteur du radical amino que peut représenter $R''_1$ peut être, par exemple, un de ceux précédemment cités pour $R_1$.

Le dérivé fonctionnel du produit de formule III' peut être l'un ceux précédemment cités pour le produit de formule III.

L'acide que l'on utilise pour obtenir le produit de formule VI à partir du produit de formule IV' est, de préférence, l'acide chlorhydrique aqueux.

La base en présence de laquelle on traite le produit de formule VI par le réactif Rd-Hal est, de préférence la triéthylamine ou la pyridine.

Les traitements des produits de formule VI et VII s'effectuent dans les conditions précédemment décrites pour les produits de formule IV.

Les produits de formule générale (I) possèdent une très bonne activité antibiotique sur les bactéries gram (+) telles que les staphylocoques, les streptocoques et, notamment, sur les staphyocoques penicillino-résistants. Leur efficacité sur les bactéries gram (−), notamment, sur les bactéries coliformes, les klebsiella, les salmonella et les proteus est particulièrement remarquable.

Ces propriétés rendent aptes lesdits produits, ainsi que leurs sels d'acides, pharmaceutiquement acceptables à être utilisés comme médicaments dans la traitement des affections à germes sensibles et, notamment, dans celui des staphylococcies, telles que septicémies à staphylocoques, staphyococcies malignes de la face ou cutanée, pyodermites, plaies septiques ou suppurantes, anthrax, phlegmons, érésipèles, staphylococcies aigües primitives ou post grippales, bronchopneumonies, suppurations pulmonaires.

Ces produits peuvent également être utilisés comme médicaments dans le traitement des colibacilloses et infections associées, dans les infections à proteus, à klebsiella et à salmonella et dans d'autres affections provoquées par des bactéries à gram (−).

La présente invention a donc également pour objet, à titre de médicaments et notamment de médicaments antibiotiques, les produits de formule (I), tels que définis ci-dessus, ainsi que leurs sels d'acides, pharmaceutiquement acceptables.

L'invention a particulièrement pour objet, à titre de médicaments et notamment de médicaments antibiotiques, les produits de formule (I) dans laquelle R représente un atome d'hydrogène ou un radical alkyle linéaire ou ramifié ayant de 1 à 4 atomes de carbone, éventuellement substitué par un radical carboxylique libre, estérifié ou salifié ou par un radical amino et Ra représente un radical alkyle ayant au plus 6 atomes de carbone, et notamment parmi ceux-ci, ceux dans laquelle Ra représente un radical méthyle et n représente le nombre 0, y compris leurs sels d'acides, pharmaceutiquement acceptables.

L'invention a également plus particulièrement pour objet, à titre de médicaments et notamment de médicaments antibiotiques, les produits de formule (I), répondant à la formule ($I_A$)

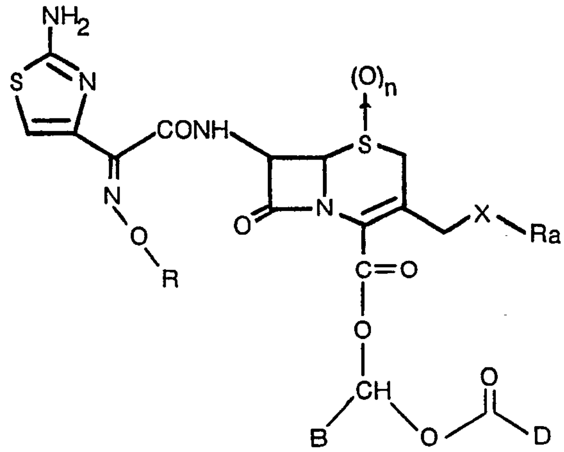

($I_A$)

dans laquelle R, n, X et Ra sont définis comme précédemment, B représente un atome d'hydrogène ou un radical alkyle linéaire ou ramifié, renfermant de 1 à 5 atomes de carbone et D représente un radical alkyle ou alkyloxy linéaire ou ramifié, renfermant de 1 à 15 atomes de carbone et notamment de 1 à 5 atomes de carbone, et, tout particulièrement parmi les produits de formule $I_A$, ceux dans lesquels B représente un atome d'hydrogène ou un radical méthyle ou éthyle et D représente un radical méthyle, éthyle, méthoxy ou éthoxy.

L'invention a également plus particulièrement pour objet, à titre de médicaments et notamment de médicaments antibiotiques, les produits décrits dans les exemples et parmi ceux-ci,

les acides 3-méthylthiométhyl 7-[[2-amino thiazol-4-yl) 2-(2-bromoethyl) oximino] acétamido] ceph-3-ème 4-carboxylique, isomère syn, et 7-[[2-(aminothiazol-4-yl)2](carboxy méthoxy imino]acétyl]amino 3-(méthyltiométhyl) ceph-3-ème 4-carboxylique isomère syn, ainsi que leurs sels avec les métaux alcalins, alcalino-terreux, le magnésium, l'ammoniaque, les bases organiques aminées, les acides et leurs esters facilement clivables.

L'invention s'étend aux compositions pharmaceutiques renfermant comme principe actif au moins un des médicaments définis ci-dessus.

Ces compositions peuvent être administrées par voie buccale, rectale, parentérale, notamment intramusculaire, ou par voie locale en application topique sur la peau et les muqueuses.

Les produits de formule (I') dans laquelle A représente un ester clivable, et notamment parmi ces produits, ceux répondant à la formule ($I_A$), et, tout particulièrement, les esters de 1-méthoxycarbonyloxyéthyle ou de 1-acétyloxyéthyle peuvent être administrés par voie orale.

Les compositions selon l'invention peuvent être solides ou liquides et se présenter sour les formes pharmaceutiques couramment utilisés en médecine humaine comme par exemple, les comprimés simples ou dragéïfiés, les gélules, les granulés, les suppositoires, les préparations injectables, les pommades, les crèmes, les gels; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent, notamment, se présenter sous forme d'une poudre destinée à être dissoute extemporanément dans un véhicule approprié, par exemple de l'eau stérile apyrogène.

La dose administrée est variable selon l'affection traitée, le sujet en cause, la voie d'administration et le produit considéré. Elle peut être, par exemple, comprise entre 0,250 g et 4 g par jour, par voie orale chez l'homme, avec le produit décrit à l'exemple 5, ou encore comprise entre 0,500 g et 1 g trois fois par jour, par voie intramusculaire.

Les produits de formule (I') peuvent également être utilisés comme désinfectants des instruments chirurgicaux.

En plus des produits décrits dans les exemples qui illustrent l'invention sans toutefois la limiter les produits suivants constituent des produits pouvant être obtenus dans le cadre de la présente invention; les substituants n, X', R'a, A et R sont ceux indiqués dans la formule (I')

| n | X′ | R′a | A | R |
|---|---|---|---|---|
| O | O | $CH_3$ | H | 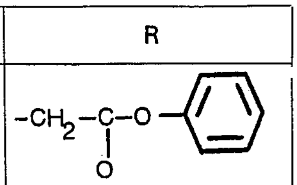 |
| O | O | $CH_3$ | H | $-CH_2-CO_2H$ |
| O | O | $CH_3$ | H | $-C(CH_3)_2CO_2O$ |
| O | O | $CH_3$ | H | $-(CH_2)_2NH_2$ |
| O | O | $CH_3$ | H | 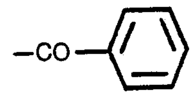 |
| O | O | $CH_3$ | H | $-COCH_3$ |
| O | O | $CH_3$ | H | $-CO_2CH_2CH_3$ |
| O | O | $CH_3$ | H | $-CON(CH_3)_2$ |
| O | O | $-CH_2-CH_3$ | H | $-CH_2-CO_2H$ |
| O | O | $-CH_2-CH_3$ | H | $-C(CH_3)_2-CO_2H$ |
| O | O | $-CH_2-CH_3$ | H | $-(CH_2)_2NH_2$ |
| O | S | $-CH_3$ | H | $-C(CH_3)_2CO_2H$ |
| O | S | $-CH_3$ | H | $-(CH_2)_2NH_2$ |
| O | S | $-CH_3$ | H | 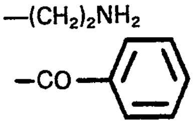 |
| O | S | $-CH_3$ | H | $-COCH_3$ |
| O | S | $-CH_3$ | H | $-CON(CH_3)_2$ |
| O | S | $-CH_3-CH_3$ | H | $-CH_2-CO_2H$ |
| O | S | $-CH_2-CH_3$ | H | $-C(CH_3)_2CO_2H$ |
| O | S | $-CH_2-CH_3$ | H | $-(CH_2)_2NH_2$ |
| O | S | $-CH_3$ | H | 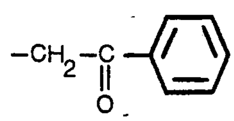 |
| O | O | $-CH_3$ | H | 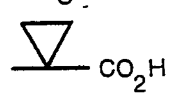 |
| O | O | $-CH_3$ | H | $-CH-CO_2H$<br>$\vert$<br>$CH_3$ |
| O | S | $-CH_3$ | H | 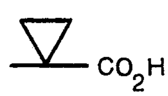 |
| O | S | $-CH_3$ | H | $-CH-CO_2H$<br>$\vert$<br>$CH_3$ |

Les produits de formule (II) sont connus ou peuvent être préparés selon les procédés indiqués dans les brevets français 2 379 540 et 2 119 074. Les produits de formules (III) et (V) sont décrits par exemple dans les brevets français 2 346 014 et 2 385 722.

Les exemples suivants illustrent l'invention sans toutefois la limiter.

Exemple de Référence 1

L'acide 3-éthoxyméthyl 7-amino ceph 3-ème 4-carboxylique utilisé peut être préparé de la façon suivante:

On agite 27,2 g d'acide 7-amino céphalosporanique et 272 $cm^3$ d'acétonitrile anhydre, ajoute 128 $cm^3$ d'éthérate de trifluorure de bore puis 71 $cm^3$ d'éthanol et chauffe à 45°—50°C pendant 16 heures sous atmosphère inerte. On refroidit le mélange réactonnel à 15—20°C, et ajoute 98 $cm^3$ de triéthylamine en 15 minutes. On essore le précipité, rince à l'acétonitrile, à l'acétone puis à l'éther éthylique pour obtenir 19,25 g de produit brut. On dissout 18 g de celui-ci dans 54 $cm^3$ d'acide chlorhydrique 2N, chauffe à 45°C, traite au charbon actif, filtre et neutralise le filtrat par addition de 11 $cm^3$ d'ammoniaque à chaud. On essore à 20°C le précipité obtenu, rince à l'eau, à l'acétone et à l'éther et obtient 8,6 g de produit. On reprend 5,86 g de ce dernier par 24 $cm^3$ d'acide chlorhydrique 2N, agite pendant 1 heure à 45°C et ajoute 4 $cm^3$ d'ammoniaque à chaud. On essore à 20°C, rince à l'eau, à l'acétone puis à l'éther éthylique et obtient 4,75 g de produit attendu.

*Spectre UV* (EtOH, HClN/10)

max: 259 nm E: 239 ε: 6.200

*Spectre RMN* (DMSO) ppm:

1,08 (t: 7=7)
3,39 (q: J=7) } —OEt
4,18 —O—$CH_2$
4,72—4,8 et 4,93—5,03 $H_6$ et $H_7$

Exemple 1

Acide 3-(2-propényloxy méthyl) 7-[2-(2-aminothiazol 4-yl) 2-méthoxyimino acétamido]ceph 3-ème 4-carboxylique isomère syn

On mélange 0,96 g d'acide 2-(2-aminothiazol 4-yl) 2-méthoxyimino acétique isomère syn, 0,912 g de chlorure de tosyl et 4 $cm^3$ de diméthylacétamide anhydre. On refroidit à −20°C et ajoute 0,67 $cm^3$ de triéthylamine en maintenant l'agitation et la température pendant 1 heurs. On ajoute en 5 minutes cette suspension à −20°C, à une solution, refroidie à −65—70°C obtenue en mélangeant 0,992 g d'acide 3-allyloxyméthyl 7-amino ceph 3-ème 4-carboxylique, 10 $cm^3$ de chlorure de méthylène et 1,5 $cm^3$ de triéthylamine à 15—20°C. On agite pendant 30 minutes à −65°C ± 2°, ajoute 2 $cm^3$ d'un mélange acide acétique-chlorure de méthylène 1—1, agite encore 15 minutes, laisse revenir à −50°C pour introduire 2 $cm^3$ d'eau. A 0°C on ajoute 4 $cm^3$ d'acide formique aqueux à 50%. On élimine le chlorure de méthylène par distillation à moins de 30°C sous pression réduite, tout en ajoutant 11 $cm^3$ d'eau. On ajoute 50 $cm^3$ d'une solution aqueuse saturée de chlorure de sodium. On décante la phase aqueuse et empâte le précipité gommeux avec 5 $cm^3$ d'eau pour concréter le produit. On essore, rince à l'eau puis à l'éther pour obtenir 0,728 g de produit brut. A la phase aqueuse décantée et aux eaux d'empâtage et de rinçage on ajoute 20 $cm^3$ d'une solution aqueuse saturée en chlorure de sodium et extrait à l'acétate de méthyle. On sèche la phase organique, concentre à sec et reprend le résidu par 20 $cm^3$ d'une solution aqueuse saturée en chlorure de sodium. On recueille le précipité, l'empâte avec 5 $cm^3$ d'eau pour obtenir encore 0,338 g de produit brut. On chromatographie sur silice 1,060 g de produit brut, en éluant par une solution 2M de chlorure de sodium renfermant 4‰ d'une solution 1M de bicarbonate de sodium. On acidifie à pH 3—4 les fractions aqueuses recueillies avec de l'acide formique aqueux à 50% essore et obtient 0,180 g de produit attendu. Du filtrat on récupère à nouveau 0,368 g de produit attendu, par extraction à l'acétate de méthyle, en opérant comme ci-dessus.

*Spectre UV* (EtOH, HCl N/10)

max: 265 nm $E_1^1$: 423 ε: 19.200

*Spectre RMN* (DMSO) ppm:

3,84: N—O—$CH_3$
3,86—3,93: O—$CH_2$—CH=
4,24: —$CH_2$O—
5,13 à 5,33: =$CH_2$ et $H_6$
5,7 à 6,1: —*CH*=$CH_2$ et $H_7$
6,72: $H_5$ du thiazole
9,5—9,6 —NHCO—

L'acide 3-allyloxyméthyl 7-amino cèph 3-ème 4-carboxylique utilisé au début de l'exemple a été préparé de la même manière que l'acide 3-éthoxy méthyl 7-amino cèph 3-ème 4-carboxylique, à l'exemple de reference 1. On emploie 27,2 g d'acide 7-amino céphalosporanique, 170 $cm^3$ d'éthérate de trifluorure de bore, 136 $cm^3$ d'alcool allylique et 115 $cm^3$ de triéthylamine. De même, on purifie le produit brut (9 g) en 2 fois successives, avec de l'acide chlorhydrique chaud et de l'ammoniaque. On obtient 4,72 g de produit attendu.

*Spectre UV* (EtOH, HCl N/10)

max: 259—260 nm $E_1^1$: =244 ε: 6.600

*Spectre RMN* (DMSO) ppm:
4.23 —$CH_2$—O—
de 5,62 à 6,25 O—*CH* = $CH_2$
4,72—4,8 et 4,95—5,03: les H du β lactame
~3,57: —$CH_2$—S—
4,23: —O—$CH_2$—
3,88: —N—O—$CH_3$
5,12—5,2: $H_6$
5,65—5,73 et 5,78—5,87: $H_7$
6,82: $H_5$ du thiazole
~9,58—9,72: —NH—CO

L'acide 3-(1-méthyl éthoxy) méthyl 7-amino cèph 3-ème 4-carboxylique utilisé au début de l'exemple a été préparé de la même manière que l'acide 3-éthoxyméthyl 7-amino cèph 3-ème-4-carboxylique à l'exemple 8. On emploie 27,2 g d'acide 7-amino céphalosporanique, 170 $cm^3$ d'éthérate de trifluorure de bore, 134 $cm^3$ d'isopropanol et 125 $cm^3$ de triéthylamine. De même on purifie le produit brut (16,25 g) en 2 fois successives, avec de l'acide chlorhydrique à chaud et de l'ammoniaque. On obtient 5,8 g de produit attendu.

*Spectre UV* (EtOH, HCl N/10)
max: 259 mm $E_1^1$: 233 ε: 6.300
*Spectre RMN* (DMSO) ppm:
1,02—1,12: 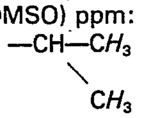
4,2: $CH_2$—O
4,72—4,8 et 4,95—5,03: $H_6$ et $H_7$.

Exemple 2

Acide 3-(phénylméthoxy) méthyl 7-[2-(2-amino thiazol 4-yl) 2-méthoxyimino acétamido]ceph 3-ème 4-carboxylique isomère syn

On opère comme à l'exemple 1 avec 1,175 g d'acide 3-(phényléthoxy) méthyl 7-amino cèph 3-ème 4-carboxylique jusqu'au stade de l'élimination du chlorure de méthylène et l'addition d'eau.

On ajoute une solution aqueuse saturée en chlorure de sodium, essore le précipité formé, rince àl'eau puis à l'éther éthylique, et obtient 0,905 g puis 0,590 g de produit. On dissout 0,707 g du produit dans 7 $cm^3$ d'eau et 0,2 $cm^3$ de triéthylamine. Après traitement un charbon actif, on ajoute 0,15 $cm^3$ d'acide formique à 50%. On essore 0,484 g de précipité, ajoute les 0,590 g de produit et triture l'ensemble dans 10 $cm^3$ d'éthanol à 50% d'eau contenant 0,6 $cm^3$ d'acide formique, pendant 15 minutes à 20°C. On essore l'insoluble et ajoute au filtrat 1,6 $cm^3$ d'ammoniaque, et 20 $cm^3$ d'eau, et extrait à l'acétate de méthyle, lave à l'eau la phase organique, sèche et concentre à sec sous pression réduite. On triture le résidu avec 10 $cm^3$ d'éthanol, essore, rince à l'éthanol puis à l'éther et obtient 0,283 g de produit attendu.

*Spectre UV*
*dans EtO*
max 237 nm $E_1^1$ 350 ε: 17.700
infl 255 nm $E_1^1$ 308
infl 290 nm $E_1^1$ 154
*dans EtOH-HCl N/10*
max 262 nm $E_1^1$ 367 ε: 18.500
*Spectre RMN* (DMSO) ppm:
3,85: —N—O—$CH_3$
4,31: —$CH_2$—O—
4,45: —O—$CH_2$—Φ
5,12—5,2: $H_6$
5,68—5,77 et 5,82—5,9: $H_7$
6,75: $H_5$ du thiazole
7,33: phényle
~9,52—9,65: —NH—COL'acide 3-(phénylméthoxy)méthyl 7-amino cèph 3-ème 4-carboxylique utilisé dans l'exemple a été préparé de la même manière que l'acide 3-éthoxyméthyl 7-amino cèph 3-ème 4-carboxylique à l'exemple de référence 1. On emploie 27,2 g d'acide 7-amino céphalosporanique, 128 $cm^3$ d'éthérate de trifluorure de bore, 126 $cm^3$ d'alcool benzylique et 90 $cm^3$ de trîthylamine, et obtient 27,1 g de produit brut.

On agite 25 g de produit brut et 250 $cm^3$ d'acide acétique ajoute 25 $cm^3$ d'éthérate de trifluorure de bore, précipite la solution par addition de 25 $cm^3$ de triethylamine. On essore le précipité, rince à l'acide acétique, à l'acétone puis à l'éther et obtient 4,6 g. On dissout à 45°C, 4,8 g produit traité comme ci-dessus, dans 20 $cm^3$ d'acide chlorhydrique 2N et 8 $cm^3$ d'acide chlorhydrique concentré; traite au charbon actif, ajoute 6 $cm^3$ d'ammoniaque. On essore le précipité formé rince à l'eau, à l'acétone et à l'éther et obtient 2 g de produit attendu.

*Spectre UV* (EtOH, HCl N/10) + DMSO
max: 259 nm $E_1^1$ 216 ε: 6.900
*Spectre RMN* (DMSO)
4,28 et 4,45: $-CH_2-O-CH_2$
4,73—4,82 et 4,95—5,03: $H_6$ et $H_7$
7,33: aromatiques.

Exemple 3

Acide 3-(2-méthoxy éthoxy) méthyl 7-[2-(2-aminothiazol 4-yl) 2-méthoxyimino acétamido]céph 3-ème 4-carboxylique isomère syn

On opère comme à l'exemple 1 avec 1,160 g d'acide 3-(2-méthoxy éthoxy) méthyl 7-amino cèph 3-ème 4-carboxylique jusqu'au stade de l'élimination du chlorure de méthylène et l'addition d'eau. On ajoute 50 $cm^3$ d'une solution aqueuse saturée de chlorure de sodium, essore l'insoluble formé et extrait le filtrat à l'acétate de méthyle. On lave la phase organique à l'eau, sèche et concentre à sec sous pression réduite. On reprend le résidu par 50 $cm^3$ d'une solution aqueuse saturée de chlorure de sodium. On élimine le précipité formé par décantation et extrait le surnageant à l'acétate de méthyle. On lave à l'eau la phase organique, sèche et concentre à sec sous pression réduite à moins de 30°C. On triture le résidu dans 20 $cm^3$ d'éther éthylique, essore et obtient 0,725 g de produit brut. On reprend 0,720 g de ce dernier dans 15 $cm^3$ d'eau, agite pendant 5 minutes à 20°C, essore un insoluble et extrait le filtrat à l'acétate de méthyle. On lave à l'eau la phase organique, sèche et concentre à sec sous pression réduite. On triture le résidu dans 20 $cm^3$ d'éther, essore et obtient 0,500 g de produit.

*Spectre UV*
*EtOH*
max 238 $E_1^1$ 342 ε = 16.100
infl 250 nm $E_1^1$ 313
infl 290 nm $E_1^1$ 141
*EtOH, HCl N/10*
max 265 nm $E_1^1$ 369 ε = 17.400
*Spectre RMN* (DMSO) ppm
3,23: $-OCH_3$
3,45: $O(CH_2)_2-O$
3,83: $N-O-CH_3$
4,28: $-CH_2-O-$
5,11—5,17: $H_6$
5,69—5,74 et 5,77—5,83: $H_7$
6,74: $H_5$ du thiazole
7,22: $NH_2$
9,5—9,6: NHCO

L'acide 3-(2-méthoxy éthoxy) méthyl 7-amino cèph 3-ème 4-carboxylique utilisé au début de l'exemple a été préparé de la même façon que l'acide 3-éthoxy méthyl 7-amino cèph 3-ème 4-carboxylique à l'exemple de reference 1. On emploie 27,2 g d'acide 7-amino céphalosporanique, 170 $cm^3$ d'éthérate de trifluorure de bore 136 $cm^3$ de méthoxy-éthanol, 125 $cm^3$ de triéthylamine et obtient 19,25 g de produit brut. On mélange 17 g de celui-ci, 170 $cm^3$ de chlorure de méthylène, et 8,3 $cm^3$ de triéthylamine, agite pendant 20 minutes, essore l'insoluble et ajoute 8,3 $cm^3$ d'acide acétique au filtrat. On essore le précipité formé, on reprend après séchage, 9,28 g dans 130 $cm^3$ d'acétone à 2% d'eau et 4,5 $cm^3$ de triéthylamine et agite pendant 15 minutes. On filtre l'insoluble et précipite le filtrat avec 4,5 $cm^3$ d'acide formique. On essore le précipité, rince à l'acétone puis à l'éther et obtient 6,35 g de produit que l'on traite à nouveau de la même façon pour obtenir 5,77 g de produit attendu.

*Spectre UV* (EtOH, HCl N/10)
max 260—261 nm $E_1^1$ 226 6.500
infl 330 nm $E_1^1$ 9
*Spectre RMN* (DMSO) ppm:
3,23: $O-CH_3$
3,45—$CH_2-S-$et $-O(CH_2)_2O-$
4,23: $-CH_2-O$
4,7—4,77 et 4,92—5: $H_6$ et $H_7$.

Stade A: Anhydride paratoluène sulfonique 2-(2-tritylamino thiazol-4-yl) 2-[(phénylméthyl) oxy imino] acétique isomère syn

On mélange à 0°C 2,6 g d'acide 2-(2-tritylamino thiazol-4-yl) 2-[(phénylméthyl) oxy imino] acétique isomère syn dans 26 $cm^3$ d'acétone, ajoute 0,95 g de chlorure de tosyle et 0,7 $cm^3$ de triéthylamine, laisse revenir à température ambiante, agite 1 heure et obtient une suspension que sera utilisée aussitôt pour le stade suivant.

Stade B: Acide 3-méthylthiométhyl 7-[2-(2-tritylamino thiazol-4-yl) 2-[(phénylméthyl) oxy imino] acétamido] ceph-3-ème 4-carboxylique isomère syn

On verse la suspension obtenue au stade A dans la solution préalablement refroidie, de 1,3 g d'acide 7-amino 3-méthylthiométhyl ceph-3-ème 4-carboxylique dans 13 $cm^3$ de chlorure de méthylène et 1,4 $cm^3$ de

triéthylamine. On laisse revenir à température ambiante, agite pendant 45 minutes, ajoute 0,7 $cm^3$ d'acide acétique puis chasse les solvants sous pression réduite. On reprend le résidu avec 40 $cm^3$ de chlorure de méthylène et 40 $cm^3$ d'acide chlorhydrique 0,1N, décante, lave la phase organique à l'eau, sèche, chasse les solvants sous pression réduite et recueille 4,44 g de résidu.

Stade C: Acide 3-méthylthiométhyl 7-[2-(2-amino thiazol-4-yl) 2-[(phénylméthyl) oxy imino] acétamido] ceph-3-ème 4 carboxylique isomère syn

On reprend le résidu par 22 $cm^3$ d'acide formique à 67%, agite 15 minutes à 45°C, ajoute 10 $cm^3$ d'eau, élimine l'insoluble, chasse les solvants sous pression réduite à 35°C, reprend le résidu par une solution aqueuse d'éthanol à 50%, puis dans l'eau, essore, sèche sous pression réduite et obtient 2,28 g de produit brut que l'on chromatographie sur silice (éluant: solution aqueuse de chlorure de sodium 3M contenant 0,4% de carbonate acide de sodium). On acidifie la phase organique par de l'acide chlorhydrique N, essore, empâte à l'eau, sèche et obtient 0,911 g de produit que l'on reprend par 8 $cm^3$ de chlorure de méthylène à 10% de méthanol, ajoute un peu de sulfate de magnésium, essore. On concentre à sec le filtrat sous pression réduite, reprend le résidu à l'éther éthylique, essore, sèche et recueille 0,686 g de produit attendu pur. F = 168°C (décomposition).

*Spectre UV* EtO
max: 236 nm ε = 18100
inf.: 257 nm
inf.: 301 nm ε = 6700
(EtOH, HCl N/10)
max.: 266 nm ε = 18800

*Spectre RMN* (DMSO) ppm
1,92: $S—CH_3$
3 à 4,16: $CH_2—S$
6,8: $H_5$ du thiazole
7,41: H du phényle
5,2: $CH_2$ Ø

L'acide 2-(2-tritylamino thiazol-4-yl) 2-[(phénylméthyl) oxyimino] acétique isomère syn utilisé au départ de l'exemple 4 peut être préparé comme suit:

a) 2-(2-tritylamino thiazol-4-yl) 2-[(phénylméthyl) oxy imino]carboxylate d'éthyle

On mélange 9,88 g de chlorhydrate du 2-(2-tritylamino thiazol-4-yl) 2-hydroxyimino carboxylate d'éthyle décrit dans le brevet français 2 383 188, 50 $cm^3$ de diméthylformamide et 13,8 g de carbonate de potassium. On ajoute en 5 minutes à 0°C, 23 $cm^3$ de chlorure de benzyle et laisse 20 heures sous agitation à température ambiante. On ajoute 500 $cm^3$ d'eau et 100 $cm^3$ d'acétate d'éthyle, décante, lave à l'eau la phase organique, sèche, et concentre sous pression réduite. Après chromatographie sur silice (éluant: cyclohexane-acétate d'éthyle 9—1), on obtient 5,51 g de produit attendu.

b) Acide 2-(2-tritylamino thiazol-4-yl) 2-[(phénylméthyl) oxy imino] acétique isomère syn

Exemple 4

Acide 3-méthylthiométhyl 7-[2-(2-amino thiazol-4-yl) 2-[(phénylméthyl) oxy imino] acétamido] ceph-3-ème 4-carboxylique isomère syn

Stade A: Anhydride paratoluène sulfonique 2-(2-tritylamino thiazol-4-yl) 2-[(phénylméthyl) oxy imino] acétique isomère syn

On mélange à 0°C 2,6 g d'acide 2-(2-tritylamino thiazol-4-yl) 2-[(phénylméthyl) oxy imino] acétique isomère syn dans 26 $cm^3$ d'acétone, ajoute 0,95 g de chlorure de tosyle et 0,7 $cm^3$ de triéthylamine, laisse revenir à température ambiante, agite 1 heure et obtient une suspension que sera utilisée aussitôt pour le stade suivant.

Stade B: Acide 3-méthylthiométhyl 7-[2-(2-tritylamino thiazol-4-yl) 2-[(phénylméthyl) oxy imino] acétamido] ceph-3-ème 4-carboxylique isomère syn

On verse la suspension obtenue au stade A dans la solution préalablement refroidie, de 1,3 g d'acide 7-amino 3-méthylthiométhyl ceph-3-ème 4-carboxylique dans 13 $cm^3$ de chlorure de méthylène et 1,4 $cm^3$ de triéthylamine. On laisse revenir à température ambiante, agite pendant 45 minutes, ajoute 0,7 $cm^3$ d'acide acétique puis chasse les solvants sous pression réduite. On reprend le résidu avec 40 $cm^3$ de chlorure de méthylène et 40 $cm^3$ d'acide chlorhydrique 0,1N, décante, lave la phase organique à l'eau, sèche, chasse les solvants sous pression réduite et recueille 4,44 g de résidu.

Stade C: Acide 3-méthylthiométhyl 7-[2-(2-amino thiazol-4-yl) 2-[(phénylméthyl) oxy imino] acétamido] ceph-3-ème 4 carboxylique isomère syn

On reprend le résidu par 22 $cm^3$ d'acide formique à 67%, agite 15 minutes à 45°C, ajoute 10 $cm^3$ d'eau, élimine l'insoluble, chasse les solvants sous pression réduite à 35°C, reprend le résidu par une solution aqueuse d'éthanol à 50%, puis dans l'eau, essore, sèche sous pression réduite et obtient 2,28 g de produit brut que l'on chromatographie sur silice (éluant: solution aqueuse de chlorure de sodium 3M contenant 0,4% de carbonate acide de sodium). On acidifie la phase organique par de l'acide chlorhydrique N, essore, empâte à l'eau, sèche et obtient 0,911 g de produit que l'on reprend par 8 $cm^3$ de chlorure de méthylène à 10% de méthanol, ajoute un peu de sulfate de magnésium, essore. On concentre à sec le filtrat sous pression réduite, reprend le résidu à l'éther éthylique, essore, sèche et recueille 0,686 g de produit attendu pur. F = 168°C (décomposition).

*Spectre UV* EtO
max: 236 nm ε = 18100
inf.: 257 nm
inf.: 301 nm ε = 6700
(EtOH, HCl N/10)
max.: 266 nm ε = 18800
*Spectre RMN* (DMSO) ppm
1,92: S—$CH_3$
3 à 4,16: $CH_2$—S
6,8: $H_5$ du thiazole
7,41: H du phényle
5,2: $CH_2$ Ø

L'acide 2-(2-tritylamino thiazol-4-yl) 2-[(phénylméthyl) oxyimino] acétique isomère syn utilisé au départ de l'exemple 4 peut être préparé comme suit:

a) 2-(2-tritylamino thiazol-4-yl) 2-[(phénylméthyl) oxy imino]carboxylate d'éthyle

On mélange 9,88 g de chlorhydrate du 2-(2-tritylamino thiazol-4-yl) 2-hydroxyimino carboxylate d'éthyle décrit dans le brevet français 2 383 188, 50 $cm^3$ de diméthylformamide et 13,8 g de carbonate de potassium. On ajoute en 5 minutes à 0°C, 23 $cm^3$ de chlorure de benzyle et laisse 20 heures sous agitation à température ambiante. On ajoute 500 $cm^3$ d'eau et 100 $cm^3$ d'acétate d'éthyle, décante, lave à l'eau la phase organique, sèche, et concentre sous pression réduite. Après chromatographie sur silice (éluant: cyclohexane-acétate d'éthyle 9—1), on obtient 5,51 g de produit attendu.

b) Acide 2-(2-tritylamino thiazol-4-yl) 2-[(phénylméthyl) oxy imino] acétique isomère syn

On mélange 5,3 g du produit obtenu au stade précédent dans 30 cm3 d'éthanol, 8 cm3 de dioxanne et 4,8 cm3 de soude 2N. On agite 20 heures à température ambiante, essore, rince par le mélange éthanol-dioxanne 4-1, puis par l'éther éthylique et recueille 4,452 g de sel de sodium. On ajoute 50 $cm^3$ d'eau, 50 $cm^3$ de chlorure de méthylène et 6 $cm^3$ d'acide chlorhydrique 2N. On décante, lave la phase organique à l'eau, sèche, chasse les solvants sous pression réduite, reprend le résidu à l'éther isopropylique et obtient après séchage 3,708 g d'acide attendu.
E ~ = 153°C.

*Spectre RMN* ($CDCl_3$) ppm
7,3: les Ø
6,5: $H_5$ du thiazole
5,25: $CH_2$—Ø

## Exemple 5

### Acide 3-méthylthiométhyl 7-[2-(2-amino thiazol-4-yl) 2-[(2-bromoéthyl) oxy imino]acétamido]ceph-3-ème 4-carboxylique isomère syn.

On opère comme à l'exemple 4 en partant de 1,909 g d'acide 2-(2-tritylamino thiazol-4-yl) 2-[(2-bromoéthyl) oxy imino]acétique isomère syn décrit dans la demande française n° 2 438 050 et 0,68 g de chlorure de tosyle, 0,927 g d'acide 7-amino 3-méthylthiométhyl ceph-3-ème 4-carboxylique et 15 $cm^3$ d'acide formique à 67%. On obtient 0,615 g de produit attendu pur.
F = 185°C (décomposition).

*Spectre UV* (EtOH)
max.: 232 nm ε = 18000
inf.: 255 nm
inf.: 296 nm ε = 7600
(EtOH, HCl N/10)
max.: 265 nm ε = 18800
inf.: 280 nm
*Spectre RMN* (DMSO) ppm
2: S—$CH_3$
3,44 à 5,2: $CH_2$—$CH_2$—Br et $CH_2$—S—$CH_3$
4,32 à 4,45: $CH_2$—$CH_2$—Br
6,9: $H_5$ du thiazole

donne 56 heures au repos. On concentre sous pression réduite et recueille 7,8 $cm^3$ de produit distillant entre 59° et 61°C (50 mm Hg).

b) carbonate monoiodé

On chauffe 1 heure 30 à 30°C 5 $cm^3$ du produit obtenu précédemment, 7,8 g d'iodure de sodium et 30 $cm^3$ d'acétone. On concentre à sec, reprend le résidu par 100 $cm^3$ d'éther et 100 $cm^3$ d'eau, agite 5 minutes, décante et extrait la phase aqueuse à l'éther. On lave la phase organique avec une solution aqueuse à l'éther. On lave la phase organique avec une solution aqueuse de métabisulfite de sodium (0,25M) puis à l'eau et enfin avec une solution aqueuse saturée en chlorure de sodium. On sèche et évapore à sec sous pression réduite (température maximum 40°C). On recueille 7,93 g de réactif que l'on utilise immédiatement.

Exemple 6

Acide 7-[[2-(2-amino thiazol-4-yl)2 [(carboxyméthoxy)imino]acétyl]amino]3-(méthylthiométhyl)ceph-3-ème 4-carboxylique isomère syn.

Stade A: Sel de triéthylamine de l'acide 7-[[2-(2-tritylamino thiazol-4-yl)2-(hydroxyimino)acétyl]amino]3-(méthylthiométhyl)ceph-3-ème 4-carboxylique isomère syn.

On met en solution 14,88 g de 7-[[2-(2-tritylamino thiazol-4-yl)2-[(1-méthyl 1-méthoxy)éthoxy imino]acétyl]amino]3-(méthylthiométhyl)ceph-3-ème 4-carboxylique isomère syn dans 60 $cm^3$ d'acétone à l'aide de 20 $cm^3$ d'acide chlorhydrique 2N. On agite 1 heure 10 minutes à température ambiante, chasse l'acétone, dilue avec 100 $cm^3$ d'eau, essore et lave à l'eau. On reprend le produit brut par 60 $cm^3$ d'acétone et 6 $cm^3$ d'eau. On ajoute 2,8 $cm^3$ de triéthylamine, amorce la cristallisation et délite par 20 $cm^3$ d'acétone, essore, empâte à l'acétone puis à l'éther. On obtient un premier jet de 11,4 g puis après reprise du résidu à l'acétone 0,85 g d'un deuxième jet, on obtient donc en tout 12,25 g de produit.

*Spectre RMN* (DMSO) ppm

1,96: $CH_3$—S—
6,66: proton en 5 du thiazole
7,36: protons du trityle

Stade B: Sel de triéthylamine de l'acide 7-[[2-(2-tritylamino thiazol-4-yl) 2-[(1,1-diméthyléthoxy)carbonylméthoxy imino]acétyl]amino]3-(méthylthiométhyl)ceph-3-ème 4-carboxylique isomère syn.

On mélange 3,09 g de sel de triéthylamine obtenus au stade A avec 30 $cm^3$ d'eau déminéralisée et 60 $cm^3$ de chlorure de méthylène, puis ajoute à 20°C 5 $cm^3$ de triéthylamine puis 4,8 g de bromacétate de tert-butyle. On agite 2 heures 30 à 20—25°C. On acidifie par 10 $cm^3$ d'acide chlorhydrique 2N, décante, lave au chlorure de méthylène puis par deux fois 100 $cm^3$ d'eau. On réextrait les eaux de lavage au chlorure de méthylène (2 fois 20 $cm^3$). On sèche les phases organiques, essore, rince et distille à sec sous vide. On empâte la résine formée à l'éther, essore, rince à l'éther et sèche sous vide à 20°C. On obtient 3 g d'acide. Le produit est repris dans un mélange benzène 7,5 $cm^3$ et triéthylamine 0,7 $cm^3$. Après dissolution on dilue par 75 $cm^3$ d'éther, essore le précipité et rince par 3 fois 2 $cm^3$ d'éther. On sèche le produit obtenu soit 2,4 g. Le produit est utilisé tel quel pour le stade suivant.

*Spectre RMN* ($CDCl_3$) ppm

2,04: $CH_3$—S—
4,76: =N—O—$CH_2$—
6,9: proton en 5 du thiazole
7,36: protons du trityle

Stade C: Acide 7-[[2-(2-amino thiazole-4-yl)2-[(carboxyméthoxy)imino. acétyl]amino]3-(méthylthiométhyl)ceph-3-ème 4-carboxylique isomère syn

On agite à 20°C pendant 25 minutes un mélange de 2,3 g de sel obtenu au stade B dans 11,5 $cm^3$ d'acide trifluoroacétique. On distille au bain d'eau la majorité de l'acide trifluoroacétique. On reprend par 100 $cm^3$ d'éther isopropylique en refroidissant. On agite 30 minutes à 20°C, essore, rince à l'éther isopropylique et sèche le produit sous vide à 20°C. On obtient 1,957 g de produit brut. On purifie le produit comme suit:

On dissout le produit brut dans un mélange de 5 $cm^3$ de carbonate acide de sodium molaire et 0,3 $cm^3$ de triéthylamine et on dilue la solution par 5 $cm^3$ d'eau salée saturée. On chromatographie sur silice en éluant par de l'eau salée (2M) à 4% de carbonate acide de sodium molaire. On précipite les élutions à pH4 par de l'acide formique à 50% d'eau. Après lavage et séchage on obtient 0,376 g de produit attendu.

Analyse: $C_{16}H_{17}O_7N_5S_3$ = 487,534

| | | | | |
|---|---|---|---|---|
| Calculé: | C% 39,42 | H% 3,51 | N% 14,36 | S% 19,73 |
| Trouvé: | 39,6 | 3,7 | 14,2 | 18,7 |

*Spectre RMN* (DMSO) ppm

2: $CH_3$—S—
4,63: N—O—$CH_2$—
6,85: proton en 5 du thiazole

Exemple 7

Acide 7-[[2-(2-amino thiazol-4-yl)2-[(éthoxycarbonyloxy)imino]acétyl]amino]3-(méthylthiométhyl)ceph-3-ème 4-carboxylique isomère syn.

Stade A: Sel de triéthylamine de l'acide 7-[[2-(2-tritylamino thiazol-4-yl)2-[(éthoxycarbonyloxy)imino]acétyl]amino]3-(méthylthiométhyl)ceph-3-ème 4-carboxylique isomère syn.

On mélange 2,32 g de sel de triéthylamine obtenu au stade A de l'exemple 6 dans un mélange de 23 $cm^3$ de chlorure de méthylène sec et 0,5 $cm^3$ de pyridine. On ajoute en 3 minutes, à température ambiante 0,6 $cm^3$ de chloroformiate d'éthyle. On abandonne 15 minutes à température ambiante. On ajoute 25 $cm^3$ d'eau contenant 6 $cm^3$ d'acide chlorhydrique normal. On décante et rince la phase aqueuse par 5 $cm^3$ de chlorure de méthylène. On réunit les phases organiques et les sèche. On essore, rince au chlorure de méthylène puis chasse le solvant. On reprend le résidu par 20 $cm^3$ d'acétate d'éthyle et agite jusqu'à dissolution totale. On ajoute 0,5 $cm^3$ de triéthylamine. Le sel précipite. On essore, rincee à l'acétate d'éthyle puis à l'éther. On sèche et obtient 1,49 g de produit.

Stade B: Acide 7-[[2-(2-amino thiazol-4-yl)2-[(éthoxycarbonyloxy)imino]acétyl]amino]3-(méthylthiométhyl)ceph-3-ème 4-carboxylique isomère syn.

On reprend la totalité du sel obtenu au stade précédent dans 7,6 $cm^3$ d'acide formique à 67%. On agite 10 minutes à 45°C. On essore le triphénylcarbinol et rince à l'acide formique à 67%. Après séchage on obtient 0,434 g de produit. Le solvant est chassé sous pression réduite à 35°C environ. Le résidu est repris à l'eau. On délite, essore puis rince à l'eau. Au produit brut obtenu, on ajoute 0,319 g de produit brut de façon identique lors d'une autre manipulation. Le produit est mis en solution dans une solution 0,5M de carbonate acide de sodium puis fixé sur 30 g de silice. On élue par de l'eau salée (3M) contenant 4% de carbonate acide de sodium. Les fractions contenant le produit attendu sont acidifiées par de l'acide acétique (pH3). Le produit purifié précipite. On abandonne 30 minutes à température ambiante puis essore et empâte quatre fois à l'eau. On sèche sous pression réduite et obtient 0,708 g de produit attendu.

Analyse: $C_{17}H_{19}O_7N_5S_3$ = 501,56

| | | | | |
|---|---|---|---|---|
| Calcule: | C% 40,7 | H% 3,8 | N% 14,0 | S% 19,2 |
| Trouvé: | 40,7 | 3,7 | 13,9 | 19,3 |

*Spectre RMN* (DMSO) ppm

1,26 (t): $CO_2$ $CH_2$—$CH_3$ J = 7Hz

2: $CH_3$—S—

4,25 (q): $CO_2$—$CH_2$—$CH_3$ J = 7Hz

7,11: proton en 5 du thiazolyle

Etude pharmacologique des produits de l'invention.

Activité in vitro, méthode des dilutions en milieu liquide.

On prépare une série de tubes dans lesquels on répartit une même quantité de milieu nutritif stérile. On distribue dans chaque tube des quantités croissantes du produit à étudier, puis chaque tube est ensemencé avec une souche bactérienne. Après incubation de vingt-quatre ou quarante-huit heures à l'étuve à 37°C, l'inhibition de la croissance est appréciée par transillumination ce qui permet de déterminer les concentrations minimales inhibitrices (C.M.I.) exprimées en µg/$cm^3$.

Les résultats suivants sont obtenus:

Produit de l'exemple 4

| SOUCHES | C.M.I. en ug/ml | |
|---|---|---|
| | 24 H | 48 H |
| Staphylococcus aureus ATCC 6 538 Pen-Sensibles | 0,5 | 0,5 |
| Staphylococcus aureus UC 1 128 Pen-Résistant | 0,5 | 0,5 |
| Staphylococcus aureus exp. n° 54 146 | 1 | 1 |
| Streptococcus pyogènes A 561 | ≤0,02 | ≤0,02 |
| Escherichia Coli Sensible Tétracycline 7624 | 3 | 5 |
| Escherichia Coli Résistant Tétracycline ATCC 11 303 | 3 | 5 |
| Escherichia Coli Exp. $TO_{26}B_6$ | 2 | 2 |
| Escherichia Coli Résistant Gentamicine, Tobramycine R 55 123 D | 3 | 3 |
| Klebsiella pneumoniae Exp. 52 145 | 0,5 | 1 |
| Klebsiella pneumoniae 2 536 Résistant Gentamycine | 10 | 10 |
| Proteus mirabilis (indol-) A 235 | 1 | 2 |
| Salmonella typhimurium 420 | 1 | 5 |

Produit de l'exemple 5

| SOUCHES | C.M.I. en ug/ml 24 H | 48 H |
|---|---|---|
| Staphylococcus aureus ATCC 6 538 Pen-Sensibles | 0,5 | 0,5 |
| Staphylococcus aureus UC 1 128 Pen-Résistant | 1 | 2 |
| Staphylococcus aureus exp. n° 54 146 | 1 | 1,5 |
| Streptococcus pyogènes A 561 | ≤0,02 | ≤0,02 |
| Escherichia Coli Sensible Tétracycline 7624 | 3 | 5 |
| Escherichia Coli Résistant Tétracycline ATCC 11 303 | 1 | 2 |
| Escherichia Coli Exp. $TO_{26}B_6$ | 3 | 3 |
| Escherichia Coli Résistant Gentamicine, Tobramycine R 55 123 D | 2 | 2 |
| Klebsiella pneumoniae Exp. 52 145 | 0,5 | 0,5 |
| Klebsiella pneumoniae 2 536 Résistant Gentamycine | 10 | 10 |
| Proteus mirabilis (indol-) A 235 | 1 | 2 |
| Salmonella typhimurium 420 | 3 | 5 |

Produit de l'exemple 6

| SOUCHES | C.M.I. en ug/ml 24 H | 48 H |
|---|---|---|
| Staphylococcus aureus ATCC 6 538 Pen-Sensibles | 3 | 5 |
| Staphylococcus aureus UC 1 128 Pen-Résistant | 5 | 5 |
| Staphylococcus aureus exp. n° 54 146 | 5 | 10 |
| Streptococcus pyogènes A 561 | 0,2 | 0,2 |
| Escherichia Coli Sensible Tétracycline 7624 | 1 | 2 |
| Escherichia Coli Résistant Tétracycline ATCC 11 303 | 0,2 | 0,5 |
| Escherichia Coli Exp. $TO_{26}B_6$ | 0,5 | 0,5 |
| Escherichia Coli Résistant Gentamicine, Tobramycine R 55 123 D | 2 | 2 |
| Klebsiella pneumoniae Exp. 52 145 | 0,2 | 0,5 |
| Klebsiella pneumoniae 2 536 Résistant Gentamycine | 1 | 1 |
| Proteus mirabilis (indol-) A 235 | 0,05 | 0,1 |
| Proteus vulgaris (indol +) A 232 | 0,1 | 0,1 |
| Salmonella typhimurium 420 | | |
| PROVIDENCIA DU 48 | 1 | 5 |
| SERRATIA RESISTANT GENTAMICINE 2 532 | 1 | 1 |

Produit de l'exemple 7

| SOUCHES | C.M.I. en ug/ml 24 H | 48 H |
|---|---|---|
| Staphylococcus aureus ATCC 6 538 Pen-Sensibles | 0,5 | 0,5 |
| Staphylococcus aureus UC 1 128 Pen-Résistant | 1 | 1 |
| Staphylococcus aureus exp. n° 54 146 | 0,5 | 1 |
| Streptococcus pyogènes A 561 | ≤0,02 | ≤0,02 |
| Streptococcus faecalis 5 432 | 10 | 10 |
| Streptococcus faecalis 99 F 74 | 3 | 10 |
| Escherichia Coli Sensible Tétracycline 7624 | 1 | 2 |
| Escherichia Coli Résistant Tétracycline ATCC 11 303 | 0,5 | 0,5 |
| Escherichia Coli Exp. $TO_{26}B_6$ | 1 | 1 |
| Escherichia Coli Résistant Gentamicine, Tobramycine R 55 123 D | 1 | 1 |
| Klebsielle pneumoniae Exp. 52 145 | 0,2 | 0,5 |
| Klebsiella pneumoniae 2 536 Résistant Gentamycine | 3 | 3 |
| Proteus mirabilis (indol-) A 235 | 0,5 | 1 |
| Salmonella typhimurium 420 | 2 | 2 |
| SERRATIA RESISTANT GENTAMICINE 2 532 | 3 | 5 |

Dans le tableau suivant, les souches utilisées sont les suivantes:

| | |
|---|---|
| Souche A | Staphylococcus aureus ATCC 6 538 Pen-Sensibles. |
| Souche B | Staphylococcus aureus UC 1 128 Pen-Résistant |
| Souche C | Staphylococcus aureus exp. n° 54 146 |
| Souche D | Streptococcus pyogènes A 561 |
| Souche E | BACILLUS subtilis ATCC 6 633 |
| Souche F | Escherichia Coli Résistant Tétracycline ATCC 11 303 |
| Souche G | Escherichia Coli Exp. TO26B6 |
| Souche H | Escherichia Coli Résistant Gentamicine Tobramycine R 55 123 D |
| Souche I | Klebsiella pneumoniae Exp. 52 145 |
| Souche J | Klebsiella pneumoniae 2 536 Résistant Gentamycine |
| Souche K | Proteus mirabilis (Indol-) A 235 |
| Souche L | Proteus vulgaris A 232 |
| Souche M | Salmonella typhimurium 420 |

Les Résultats sont exprimés en CMI après 24 heures d'incubation.

| Souches / Produits des exemples | A | B | C | D | E | F | G | H | I | J | K | L | M |
|---|---|---|---|---|---|---|---|---|---|---|---|---|---|
| 1 | 2 | 2 | 2 | ≤0,02 | 3 | 1 | 1 | 2 | 0,5 | 3 | 0,2 | 10 | 2 |
| 2 | 1 | 2 | 1 | ≤0,02 | 1 | 2 | 5 | 5 | 2 | 10 | 1 | — | 3 |
| 3 | 3 | 10 | 3 | 0,05 | 2 | 0,5 | 1 | 1 | 0,2 | 3 | 0,2 | 2 | 2 |

**Revendications pour les Etats contractants: BE CH LI DE GB IT LU NL SE**

1. Les produits de formule générale (I):

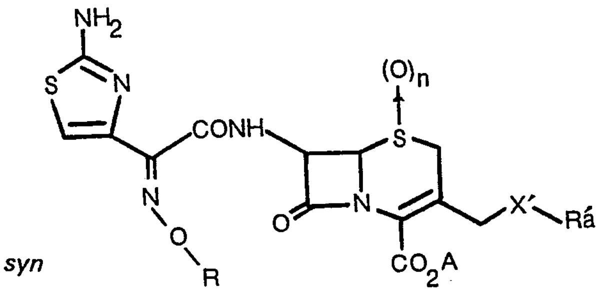

(I′)

isomères *syn*

dans laquele R, A, X′, R′a, n ont les significations suivantes *ou bien* A] R représente,

*soit* un radical alkyle linéaire ou ramifié, alkényle, alkynyle ou cycloalkyle ayant au plus 6 atomes de carbone,

*soit* un radical acétyle, propionyle, butyryle, valéryle, hexanoyle, acryloyle, crotonoyle, benzoyle, carbamoyle, chacun de ces radicaux étant éventuellement substitué par un ou plusieurs radicaux choisis parmi les radicaux carboxy éventuellement salifié ou estérifié, méthoxy carboxyle, éthoxy carbonyle, carbamoyle, diméthylcarbamoyle amino, méthylamino, diméthylamino, halogène, méthoxy, éthoxy, propyloxy, méthylthio, éthylthio phényle, tétrazolyle, phénylthio, tétrazolylthio ou thiadiazolylthio éventuellement substitué par méthyle,

*soit* R représente un radical méthoxy carbonyle ou éthoxycarbonyle.

A représente un atome d'hydrogène, un équivalent de métal alcalin, alcalino-terreux, de magnésium, d'ammonium ou d'une base organique aminée, ou A représente le reste d'un groupement ester facilement clivable,

R′a représente un radical alkyle éventuellement interrompu par un hétéroatome, alkényle ou alkynyle ayant au plus 6 atomes de carbone linéaire ou ramifié, ou un radical benzyle ou phénylethyle éventuellement substitué par un radical carboxy, amino ou diméthylaminoéthyle.

n est égal à 0, 1 ou 2,

X′ représente un atome de soufre éventuellement oxydé sous forme de sulfoxyde ou de sulfone, ou un atome d'oxygène, étant entendu que R ne représente pas un radical alkényle ou alkynyle ayant au plus 6 atomes de carbone ou un radical alkyle linéaire ou ramifié ayant au plus 6 atomes de carbone éventuellement substitué par ou plusieurs radicaux choisis parmi les radicaux carboxy éventuellement salifié ou estérifié, alcoxycarbonyle, amino, alkylamino, dialkylamino ou aryle hétérocyclique, lorsque le radical —X′—R′a représente un radical alkoxy, alkylthio ou alkénylthio.

*Ou bien* B] n est égal à 0, A représente un atome d'hydrogène et

*soit* R représente un radical $—CH_2—CH_2—NH_2$ $—CH_2CO_2H$ ou $—C(CH_3)_2CO_2H$, X′ représente un atome d'oxygène ou de soufre et R′ a représente un radical méthyle ou éthyle

*soit* R représente un radical

$$\underset{\displaystyle CH_3}{—\underset{|}{CH}—CO_2H,}$$

X′ représente un atome d'oxygène ou de soufre et R′a représente un radical méthyle ainsi que les sels des produits de formule (I′) avec les acides minéraux ou organiques.

2. Les produits selon la revendication 1, répondant à la formule générale (I)

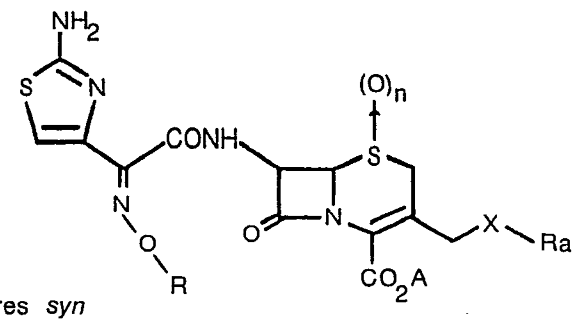

(I)

isomères *syn*

dans laquelle R, A et n sont définis comme à la revendication 1, Ra représente un radical alkyle, alkényle ou alkynyle ayant au plus 6 atomes de carbone linéaire ou ramifié, ou un radical benzyle ou thénylethyle éventuellement substitué par un radical carboxy, amino, ou diméthyl aminoethyle et X représente un atome de soufre ou un atome d'oxygène, ainsi que les sels des produits de formule (I) avec les acides minéraux ou organiques.

3. Les produits de formule générale (I) telle que définie à la revendication 2, dans laquelle R représente un radical alkyle linéaire ou ramifié ayant de 1 à 4 atomes de carbone, substitué par un radical carboxylique libre, estérifié ou salifié, ou par un radical amino et Ra représente un radical alkyle ayant au plus 6 atomes de carbone.

4. Les produits de formule générale (I) telle que définie à la revendication 2 ou 3, dans laquelle Ra représente un radical méthyle et n représente le nombre 0.

5. Les produits selon la revendication 2, répondant à la formule générale ($I_A$):

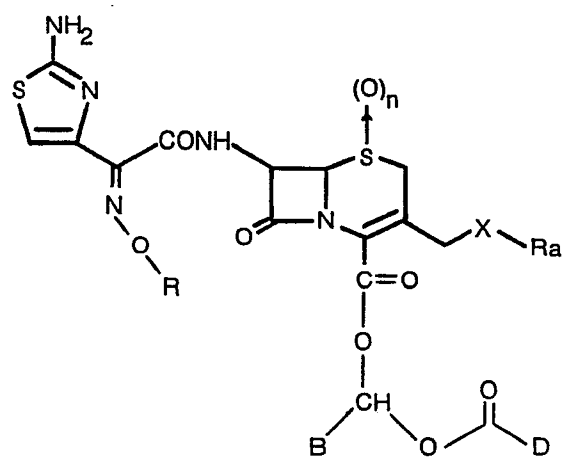

($I_A$)

dans laquelle R, n, X et Ra sont définis comme à la revendication 2, B représente un atome d'hydrogène ou un radical alkyle linéaire ou ramifié, renfermant de 1 à 5 atomes de carbone et D représente un radical alkyle ou alkoxy linéaire ou ramifié, renfermant de 1 à 15 atomes de carbone ainsi que leurs sels avec les acides minéraux ou organiques.

6. Les produits selon la revendication 5, caractérisé en ce que D représente un radical alkyle ou alkoxy, linéaire ou ramifié, renfermant de 1 à 5 atomes de carbone.

7. Les acides 3-méthylthiométhyl 7-[[2-(2-amino thiazol-4-yl)2-(2-bromoethyl)oximino]acétamido]-ceph-3-ème 4-carboxylique, isomère syn, et 7-[[2-(aminothiazol-4-yl)2](carboxy méthoxy imino]acétyl]amino 3-(méthyltiométhyl)ceph-3-ème 4-carboxylique isomère syn, ainsi que leurs sels avec les métaux alcalins, alcalino-terreux, le magnésium, l'ammoniaque, les bases organiques aminées, les acides et leurs esters facilement clivables.

8. Procédé de préparation des produits de formule (I') telle que définie à la revendication 1, caractérisé en ce que l'on traite un produit de formule (II):

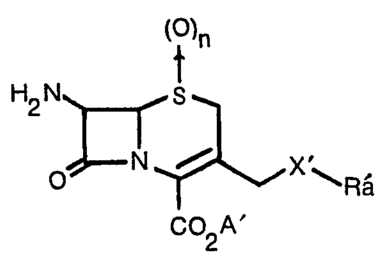

(II)

dans laquelle n, X' et R'a ont la signification indiquée à la revendication 1 et A' représente un atome d'hydrogène ou le reste d'un groupement ester facilement éliminable, par un produit de formule (III)

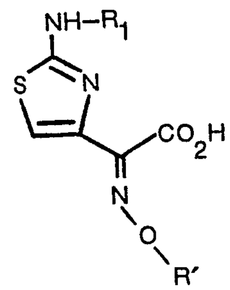

(III)

ou un dérivé fonctionnel de cet acide, formule (III) dans laquelle $R_1$ représente un atome d'hydrogène ou un groupement protecteur du radical amino et soit R' représente un radical alkyle linéaire ou ramifié, alkényle, alkynyle ou cycloalkyle ayant au plus 6 atomes de carbone, soit R' représente un radical acétyle, propionyle, butyryle, valéryle, hexanoyle, acryloyle, crotonyle, benzoyle, carbamoyle, chacun de ces radicaux étant éventuellement substitué par un ou plusieurs radicaux choisis parmi les radicaux carboxy éventuellement salifié ou estérifié, alkoxycarbonyle, carbamoyle, diméthylcarbamoyle, amino, alkylamino, dialkylamino, halogène, alkoxy, alkylthio, aryle, aryle hétérocyclique, thio éventuellement substitué par alkyle

*soit* R représente un radical alkoxycarbonyle pour obtenir un produit de formule (IV):

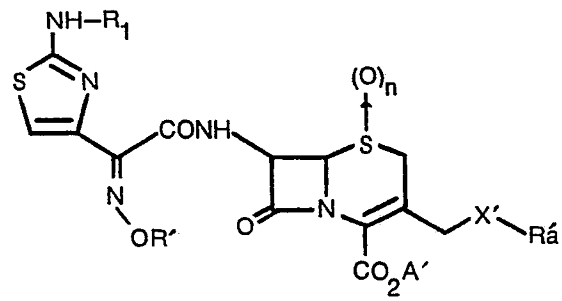

(IV)

dans laquelle $R_1$, R', A', R'a, X' et n ont la signification précédente, produit que, si désiré, dans le cas où n est égal à O et X' représente un atome de soufre ou d'oxygène, l'on traite par un agent d'oxydation, pour obtenir un produit de formule (IV) dans laquelle n est égal à 1 ou 2 et X' représente un atome de soufre oxydé sous forme de sulfoxyde ou de sulfone ou un atome d'oxygène et, produit de formule IV que l'on soumet si nécessaire ou si désiré à l'une ou plusieurs des réactions suivantes, dans un ordre quelconque:

a) coupure par hydrolyse, hydrogénolyse ou par action de la thiourée de tout ou partie des groupements esters ou des groupements de protection du ou des radicaux amino,

b) estérification ou salification par une base du ou des radicaux carboxyliques,

c) salification par un acide du ou des radicaux amino.

9. Procédé de préparation des produits de formule (I') telle que définie à la revendication 1, caractérisé en ce que l'on traite un produit de formule (V):

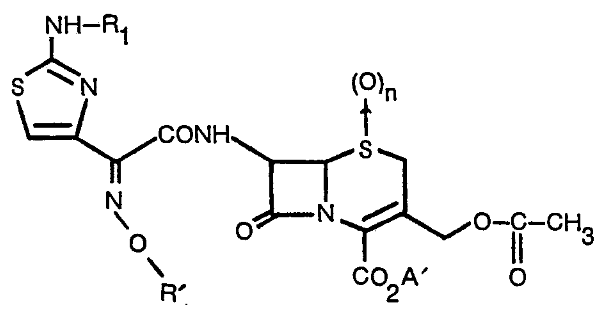

(V)

dans laquelle $R_1$, R', A' et n ont la signification indiquée à la revendication 8, *ou bien* par un produit de formule R'a-SH dans laquelle R'a a la signification indiquée à la revendication 1, *ou bien* d'abord par le 2-mercapto pyridine N-oxyde puis par un produit de formule R'aOH dans laquelle R'a a la signification précédente, pour obtenir un produit de formule (IV), tel que défini précédemment, produit de formule IV que, si désiré, dans le cas où n est égal à O et X' représente un atome de soufre ou d'oxygène, l'on traite par un agent d'oxydation, pour obtenir un produit de formule (IV) dans laquelle n est égal à 1 ou 2 et X'

représente un atome de soufre oxydé sous forme de sulfoxyde ou de sulfone ou un atome d'oxygène et, produit de formule IV que l'on soumet si nécessaire ou si désiré, à l'une ou plusieurs des réactions suivantes, dans un ordre quelconque:

a) coupure par hydrolyse, hydrogénolyse ou par action de la thiourée de tout ou partie des groupements esters ou des groupements de protection du ou des radicaux amino,

b) estérification ou salification par une base du ou des radicaux carboxyliques,

c) salification par un acide du ou des radicaux amino.

10. Procédé selon la revendication 8 de préparation des produits de formule (I'), caractérisé en ce que l'on utilise, pour la mise en oeuvre du procédé, un produit de formule (III) dans laquelle $R_1$ représente un groupement protecteur du radical amino et en ce que le dérivé fonctionnel de l'acide de formule (III) est un anhydride mixte carboxylique sulfonique.

11. Procédé de préparation des produits de formule (I'), telle que définie à la revendication 1, caractérisé en ce que l'on traite un produit de formule (II'):

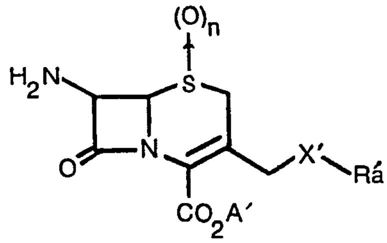

(II')

dans laquelle R'a, n, X' et A' ont la signification indiquée à la revendication 1, par un produit de formule (III'):

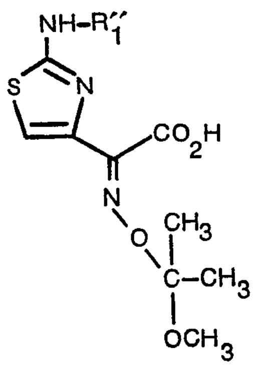

(III')

ou un dérivé fonctionnel de cet acide, formule (III') dans laquelle $R''_1$ représente un groupement protecteur du radical amino, pour obtenir un produit de formule (IV'):

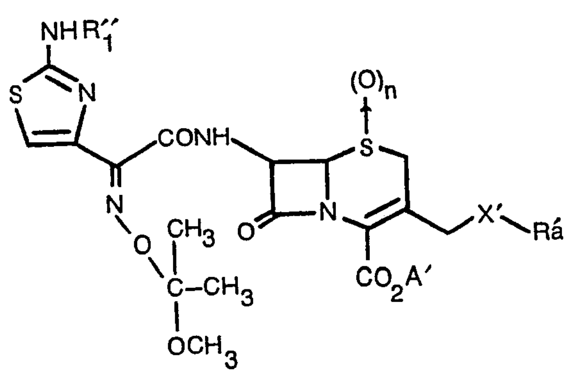

(IV')

dans laquelle A', $R''_1$, R'a, X' et n ont la signification précédente, produit de formule (IV') que l'on traite par un acide dans des conditions modérées, pour obtenir un produit de formule VI:

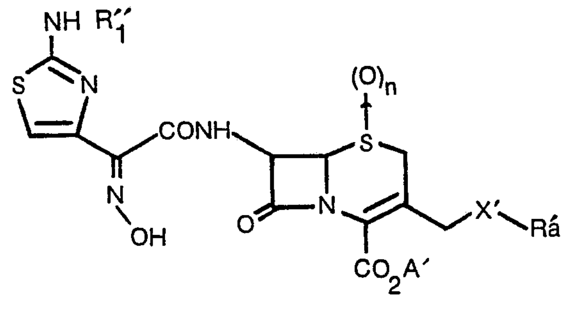

(VI)

produit que, si désiré, l'on estérifie ou salifie et traite en présence d'une base par un dérivé fonctionnel de formule:

Rd-Hal

dans laquelle Rd représente soit un radical alkyle linéaire ou ramifié, alkényle, alkynyle ou cycloalkyle ayant au plus 6 atomes de carbone, *soit* un radical acétyle, propionyle, butyryle, valéryle, hexanoyle, acryloyle, crotonoyle, benzoyle, carbamoyle, chacun de ces radicaux étant éventuellement substitué par un ou plusieurs radicaux choisis parmi les radicaux carboxy éventuellement salifié ou estérifié, alkoxycarbonyle, carbamoyle, diméthylcarbamoyle, amino, alkylamino, dialkylamino, halogène, alkoxy, alkylthio, aryle, aryle hétérocyclique, arylthio, aryle hétérocyclique, thio éventuellement substitué par alkyle *soit* Rd représente un radical alkoxycarbonyle et Hal représente un atome d'halogène, pour obtenir un produit de formule VII:

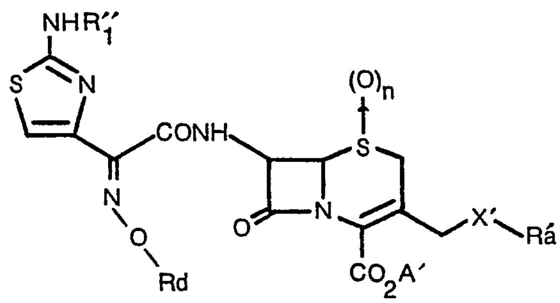

(VII)

dans laquelle $R''_1$, R'a, Rd, A', X' et n ont la signification indiquée ci-dessus et produit de formule VII que l'on soumet à une hydrolyse, hydrogénolyse ou à l'action de la thiourée pour éliminer le radical $R''_1$ de protection du radical amino et, si désiré, ou si nécessaire, à l'une ou plusieurs des réactions suivantes, dans un ordre quelconque:

a) élimination du ou des groupements esters,

b) estérification ou salification par une base du ou des radicaux carboxyliques,

c) salification par un acide du ou des radicaux amino.

12. A titre de médicaments, les produits répondant à la formule (I') telle que définie à la revendication 1, ainsi que leurs sels d'acides, pharmaceutiquement acceptables.

13. A titre de médicaments, les produits de formule (I) telle que définie à l'une quelconque des revendications 2, 3 et 4 ainsi que leurs sels d'acides, pharmaceutiquement acceptables.

14. A titre de médicaments, les produits tels que définis à la revendication 5 ou 6, ainsi que leurs sels d'acides, pharmaceutiquement acceptables.

15. A titre de médicaments, les produits tels que définis à la revendication 7, ainsi que leurs sels pharmaceutiquement acceptables.

16. Compositions pharmaceutiques contenant, à titre de principe actif, au moins un des médicaments selon l'une des revendications 12 à 15.

**Revendications pour l'Etat contractant: AT**

1. Procédé pour préparer les produits de formule générale (I')

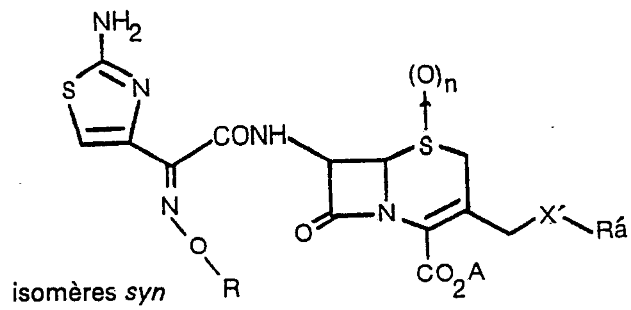

isomères *syn*

(I')

dans laquelle R, A, X', R'a, n ont les significations suivantes *ou bien:*

A] R représente,

*soit* un radical alkyle linéaire ou ramifié, alkényle, alkynyle ou cycloalkyle ayant au plus 6 atomes de carbone,

*soit* un radical acétyle, propionyle, butyryle, valéryle, hexanoyle, acryloyle, crotonoyle, benzoyle, carbamoyle, chacun de ces radicaux étant éventuellement substitué par un ou plusieurs radicaux choisis parmi les radicaux carboxy éventuellement salifié ou estérifié, méthoxy carboxyle, éthoxy carbonyle, carbamoyle, diméthylcarbamoyle, amino, méthylamino, diméthylamino, halogène, méthoxy, éthoxy, propyloxy, méthylthio, éthylthio phényle, tétrazolyle, phénylthio, tétrazolylthio ou thiadiazolylthio éventuellement substitué par méthyle,

*soit* R représente un radical méthoxy carbonyle ou éthoxycarbonyle.

A représente un atome d'hydrogène, un équivalent de métal alcalin, alcalinoterreux, de magnésium, d'ammonium ou d'une base organique aminée, ou A représente le reste d'un groupement ester facilement clivable,

R'a représente un radical alkyle éventuellement interrompu par un hétéroatome, alkényle ou alkynyle ayant au plus 6 atomes de carbone linéaire ou ramifiée, ou un radical benzyle ou phényléthyle éventuellement substitué par un radical carboxy, amino ou diméthylaminoéthyle.

n est égal à 0, 1 ou 2,

X' représente un atome de soufre éventuellement oxydé sous forme de sulfoxyde ou de sulfone, ou un atome d'oxygène, étant entendu que R ne représente pas un radical alkényle ou alkynyle ayant au plus 6 atomes de carbone ou un radical alkyle linéaire ou ramifié ayant au plus 6 atomes de carbone éventuellement substitué par ou plusieurs radicaux choisis parmi les radicaux carboxy éventuellement salifié ou estérifié, alcoxycarbonyle, amino, alkylamino, dialkylamino ou aryle hétérocyclique, lorsque le radical —X'—R'a représente un radical alkoxy, alkylthio ou alkénylthio.

*Ou bien* B] n est égal à 0, A représente un atome d'hydrogène et

*soit* R représente un radical $CH_2—CH_2NH_2$, $—CH_2CO_2H$ ou $—C(CH_3)_2—CO_2H$, X' représente un atome d'oxygène ou de soufre et R' a représente un radical méthyle ou éthyle

*soit* R représente un radical

$$\begin{array}{l}—CH—CO_2H,\\ \quad\;\, |\\ \quad CH_3\end{array}$$

X' représente un atome d'oxygène ou de soufre et R'a représente un radical méthyle ainsi que les sels des produits de formule (I') avec les acides minéraux ou organiques caractérisé en ce que l'on traite un produit de formule (II)

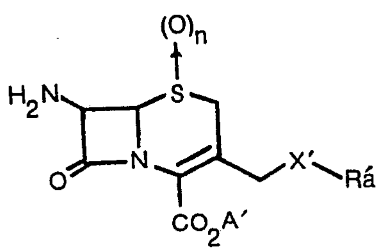

(II)

dans laquelle n, X' et R'a ont la signification indiquée, précédemment et A' représente un atome d'hydrogène ou le reste d'un groupement ester facilement éliminable, par un produit de formule (III):

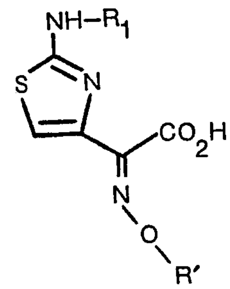

(III)

ou un dérivé fonctionnel de cet acide, formule (III) dans laquelle $R_1$ représente un atome d'hydrogène ou un groupement protecteur du radical amino et soit R' représente un radical alkyle linéaire ou ramifié, alkényle, alkynyle ou cycloalkyle ayant au plus 6 atomes de carbone, soit R' représente un radical acétyle, propionyle, butyryle, valéryle, hexanoyle, acryloyle, crotonyle, benzoyle, carbamoyle, ou alcoxycarbonyle, chacun de ces radicaux étant éventuellement substitué, pour obtenir un produit de formule (IV):

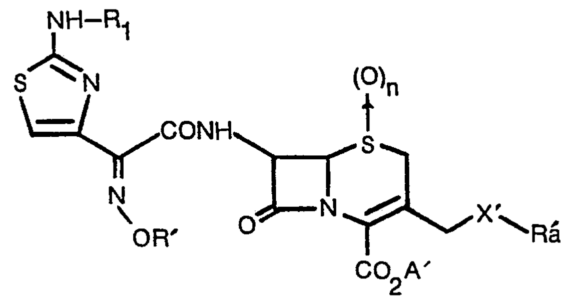

(IV)

dans laquelle $R_1$, R', A', R'a, X' et n ont la signification précédente, produit que, si désiré, dans le cas où n est égal à O et X' représente un atome de soufre ou d'oxygène, l'on traite par un agent d'oxydation, pour obtenir un produit de formule (IV) dans laquelle n est égal à 1 ou 2 et X' représente un atome de soufre oxydé sous forme de sulfoxyde ou de sulfone ou un atome d'oxygène et, produit de formule IV que l'on soumet si nécessaire ou si désiré à l'une ou plusieurs des réactions suivantes, dans un ordre quelconque:

a) coupure par hydrolyse, hydrogénolyse ou par action de la thiourée de tout ou partie des groupements esters ou des groupements de protection du ou des radicaux amino,

b) estérification ou salification par une base du ou des radicaux carboxyliques,

c) salification par un acide du ou des radicaux amino.

2. Procédé de préparation des produits de formule (I') telle que définie à la revendication 1, caractérisé en ce que l'on traite un produit de formule (V):

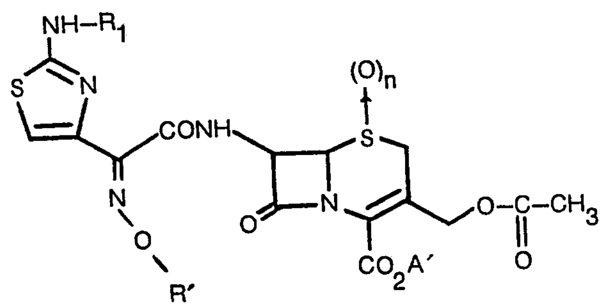

(V)

dans laquelle $R_1$, R', A' et n ont la signification indiquée à la revendication 1, *ou bien* par un produit de formule R'a-SH dans laquelle R'a a la signification indiquée à la revendication 1, *ou bien* d'abord par le 2-mercapto pyridine N-oxyde puis par un produit de formule R'aOH dans laquelle R'a a la signification précédente, pour obtenir un produit de formule (IV), tel que défini précédemment, produit de formule IV que, si désiré, dans le cas où n est égal à O et X' représente un atome de soufre ou d'oxygène, l'on traite par un agent d'oxydation, pour obtenir un produit de formule (IV) dans laquelle n est égal à 1 ou 2 et X' représente un atome de soufre oxydé sous forme de sulfoxyde ou de sulfone ou un atome d'oxygène et, produit de formule IV que l'on soumet si nécessaire ou si désiré, à l'une ou plusieurs des réactions suivantes, dans un ordre quelconque:

a) coupure par hydrolyse, hydrogénolyse ou par action de la thiourée de tout ou partie des

groupements esters ou des groupements de protection du ou des radicaux amino,

b) estérification ou salification par une base du ou des radicaux carboxyliques,

c) salification par un acide du ou des radicaux amino.

3. Procédé selon la revendication 1 de préparation des produits de formule 'I'), caractérisé en ce que l'on utilise, pour la mise en oeuvre du procédé, un produit de formule (III) dans laquelle $R_1$ représente un groupement protecteur du radical amino et en ce que le dérivé fonctionnel de l'acide de formule (III) est un anhydride mixte carboxylique sulfonique.

4. Procédé selon l'une quelconque des revendications 1, 2 et 3, pour préparer les produits selon la revendication 1, répondant à la formule générale (I):

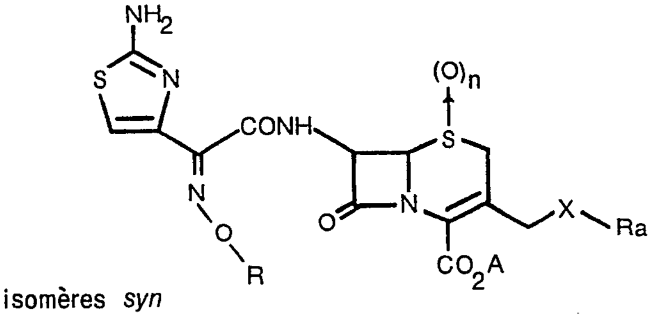

isomères *syn*

(I)

dans laquelle R, A et n sont définis comme à la revendication 1, Ra représente un radical alkyle, alkényle ou alkynyle ayant au plus 6 atomes de carbone linéaire ou ramifié, ou un radical aralkyle éventuellement substitué et X représente un atome de soufre ou un atome d'oxygène, ainsi que les sels des produits de formule (I) avec les acides minéraux ou organiques, caractérisé en ce que l'on utilise au départ des produits de formule (II), R'aSH et R'aOH dans lesquelles X' et R'a ont les valeurs précédemment indiquées pour X et Ra.

5. Procédé de préparation des produits de formule (I'), telle que définie à la revendication 1, caractérisé en ce que l'on traite un produit de formule (II'):

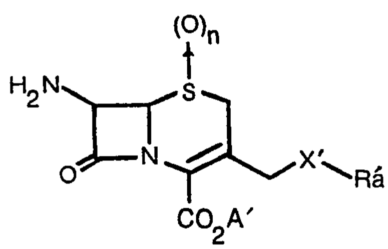

(II')

dans laquelle R'a, n, X' et A' ont la signification indiquée à la revendication 1, par un produit de formule (III'):

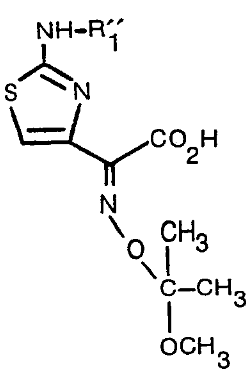

(III')

ou un dérivé fonctionnel de cet acide, formule (III') dans laquelle $R''_1$ représente un groupement protecteur du radical amino, pour obtenir un produit de formule (IV'):

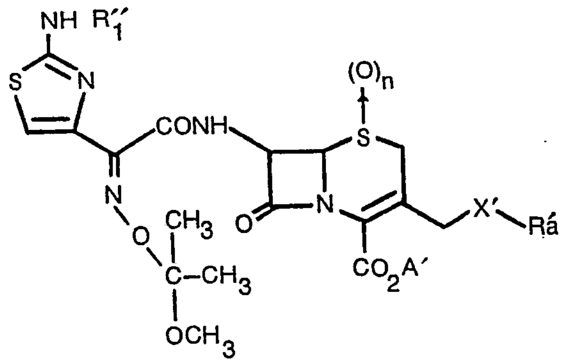

(IV')

dans laquelle A', $R''_1$, R'a, X' et n ont la signification précédente, produit de formule (IV') que l'on traite par un acide dans des conditions modérées, pour obtenir un produit de formule VI:

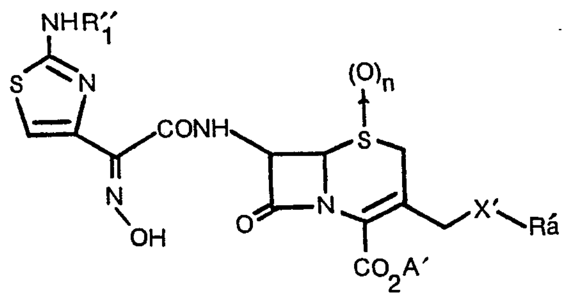

(VI)

produit que, si désiré, l'on estérifie ou salifie et traite en présence d'une base par un dérivé fonctionnel de formule:

Rd-Hal

dans laquelle Rd représente un radical alkyle linéaire ou ramifié, alkényle, alkynyle ou cycloalkyle ayant au plus 6 atomes de carbone, un radical acétyle, propionyle, butyryle, valéryle, hexanoyle, acryloyle, crotonoyle, benzoyle, carbamoyle ou alcoxycarbonyle, chacun de ces radicaux étant éventuellement substitué et Hal représente un atome d'halogène, pour obtenir un produit de formule VII:

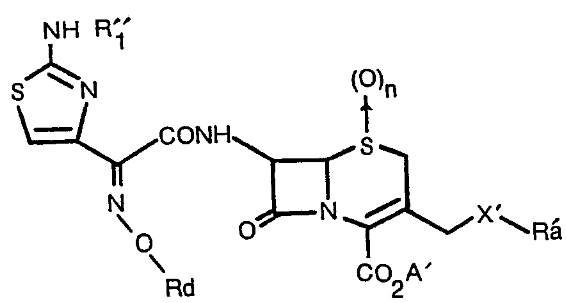

(VII)

dans laquelle $R''_1$, R'a, Rd, A', X' et n ont la signification indiquée ci-dessus et produit de formule VII que l'on soumet à une hydrolyse, hydrogénolyse ou à l'action de la thiourée pour éliminer le radical $R''_1$ de protection du radical amino et, si désiré, ou si nécessaire, à l'une ou plusieurs des réactions suivantes, dans un ordre quelconque:

a) élimination du ou des groupements esters,

b) estérification ou salification par une base du ou des radicaux carboxyliques,

c) salification par un acide du ou des radicaux amino.

6. Procédé selon la revendication 4, caractérisé en ce que dans le produit de formule (III), R' représente un radical alkyle linéaire ou ramifié, ayant de 1 à 4 atomes de carbone, éventuellement substitué par un radical carboxylique libre ou estérifié ou par un radical amino et en ce que dans le produit de formule (II) ou

dans le produit de formule R'aSH ou R'aOH, R'a représente un radical alkyle ayant au plus 6 atomes de carbone.

7. Procédé selon la revendication 5, caractérisé en ce que R'a représente un radical méthyle et en ce que dans le produit de formule (II), n est égal à 0.

8. Procédé selon la revendication 4, caractérisé en ce que l'estérification finale éventuelle est effectuée par un dérivé fonctionnel du groupement de formule

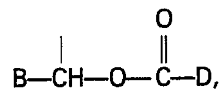

dans lequel B représente un atome d'hydrogène ou un radical alcoyle linéaire ou ramifié, renfermant de 1 à 5 atomes de carbone et D représente un radical alcoyle ou alkoxy linéaire ou ramifié, renfermant de 1 à 15 atomes de carbone.

9. Procédé selon la revendication 7, caractérisé en ce que D représente un radical alcoyle ou alkoxy, linéaire ou ramifié, renfermant de 1 à 5 atomes de carbone.

10. Procédé de préparation de produits de formule (I'), tels qu'obtenus à l'une quelconque des revendications 6 à 9, caractérisé en ce que l'on opère selon le procédé de la revendication 5.

11. Procédé selon la revendication 4 ou 10, caractérisé en ce que l'on prépare le produit répondant à la formule (I) suivante: — l'acide 3-méthylthiométhyl 7-[[2-(2-amino thiazol-4-yl) 2-(2-bromoéthyl) oxyimino acétamido ceph-3-ème 4-carboxylique, isomère syn, ainsi que ses sels avec les métaux alcalins, alcalino-terreux, le magnésium, l'ammoniaque, les bases organiques aminées, les acides et ses esters facilement clivables.

**Patentansprüche für die Vertragsstaaten: BE CH DE GB IT LI LU NL SE**

1. Verbindungen der allgemeinen Formel (I'),

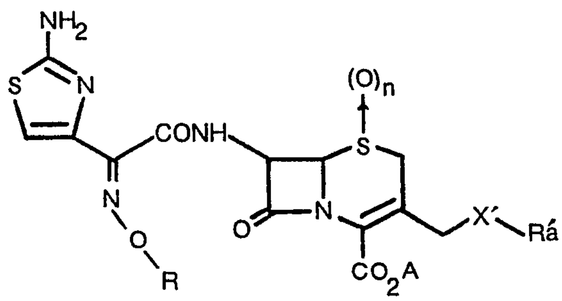

(I')

Syn-Isomere in der R, A, X', R'a und n die folgenden Bedeutungen haben oder

A] R bedeutet entweder geradkettiges oder verzweigtes Alkyl, Alkenyl, Alkinyl oder Cycloalkyl mit höchstens 6 Kohlenstoffatomen, oder Acetyl, Propionyl, Butyryl, Valeryl, Hexanoyl, Acryloyl, Crotonoyl, Benzoyl, Carbamoyl, wobei jeder dieser Reste gegebenenfalls substituiert ist mit einem oder mehreren Resten, ausgewählt unter Carboxyl, gegebenenfalls als Salz oder Ester, Methoxycarbonyl, Ethoxycarbonyl, Carbamoyl, Dimethylcarbamoyl, Amino, Methylamino, Dimethylamino, Halogen, Methoxy, Ethoxy, Propyloxy, Methylthio, Ethylthiophenyl, Tetrazolyl, Phenylthio, Tetrazolylthio oder Thiadiazolylthio,' gegebenenfalls mit Methyl substituiert, oder Methoxycarbonyl oder Ethoxycarbonyl

A bedeutet: Wasserstoff, Alkalimetall, Erdalkalimetall, Magnesium, Ammonium oder eine organische Aminobase oder einen leicht abspaltbaren Esterrest,

R'a bedeutet: geradkettiges oder verzweigtes Alkyl, gegebenenfalls unterbrochen durch ein Heteroatom, Alkenyl oder Alkinyl mit höchstens 6 Kohlenstoffatomen, Benzyl oder Phenylethyl, gegebenenfalls substituiert mit Carboxyl, Amino oder Dimethylaminoethyl,

n 0, 1 oder 2 ist,

X' gegebenenfalls oxidierten Schwefel in Form von Sulfoxyd oder Sulfon oder Sauerstoff bedeutet,

wobei R nicht Alkenyl oder Alkinyl mit höchsten 6 Kohlenstoffatomen oder geradkettiges oder verzweigtes Alkyl mit höchstens 6 Kohlenstoffatomen, gegebenenfalls substituiert mit einem oder mehreren Resten, ausgewählt unter Carboxyl, gegebenenfalls als Salz oder Ester, Alkoxycarbonyl, Amino, Alkylamino, Dialkylamino heterozyklisches Aryl, bedeutet, wenn der Rest —X'—R'a Alkoxy, Alkylthio oder Alkenylthio ist, oder

B] n 0 ist,

A Wasserstoff bedeutet und

R entweder $—CH_2—CH_2—NH_2$, $—CH_2CO_2H$ oder $—C(CH_3)_2\ CO_2H$ bedeutet,

X' Sauerstoff oder Schwefel und
R'a Methyl oder Ethyl ist, oder

$R—CH—CO_2H$ ist,
|
$CH_3$

X' Sauerstoff oder Schwefel und
R'a Methyl bedeutet, sowie die Salze der Verbindungen der Formel (I') mit anorganischen oder organischen Säuren.

2. Verbindungen nach Anspruch 1 der allgemeinen Formel (I)

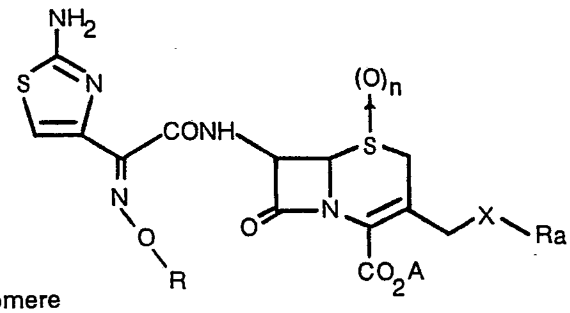

(I)

Syn-Isomere

in der R, A und n wie in Anspruch 1 definiert sind, Ra geradkettiges oder verzweigtes Alkyl, Alkenyl oder Alkinyl mit höchstens 6 Kohlenstoffatomen oder Benzyl oder Phenylethyl ist, das gegebenenfalls mit Carboxyl, Amino oder Dimethylaminoethyl substituiert ist, und X Schwefel oder Sauerstoff bedeutet, sowie die Salze der Verbindungen der Formel (I) mit anorganischen oder organischen Säuren.

3. Verbindungen der allgemeinen Formel (I) nach Anspruch 2, in der R geradkettiges oder verzweigtes Alkyl mit 1 bis 4 Kohlenstoffatomen, gegebenenfalls substituiert mit einer freien oder veresterten Carboxylgruppe oder deren Salze oder einer Aminogruppe, und Ra Alkyl mit höchstens 6 Kohlenstoffatomen ist.

4. Verbindungen der algemeinen Formel (I) nach Anspruch 2 oder 3, in der Ra Methyl und n 0 ist.

5. Verbindungen nach Anspruch 2 der allgemeinen Formel ($I_A$),

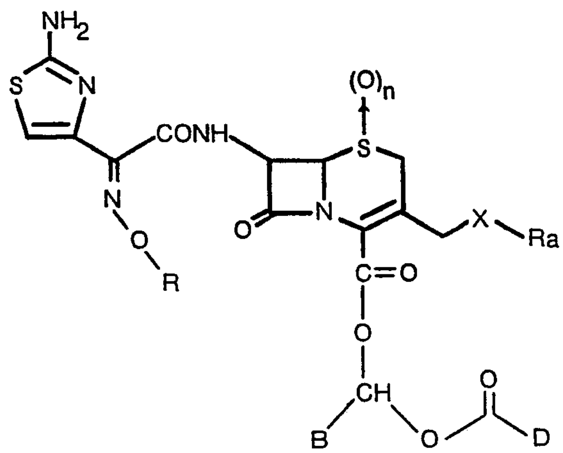

($I_A$)

in der R, n, X und Ra wie in Anspruch 2 definiert sind, B Wasserstoff oder geradkettiges oder verzweigtes Alkyl mit 1 bis 5 Kohlenstoffatomen ist und D geradkettiges oder verzweigtes Alkyl oder Alkoxy mit 1 bis 15 Kohlenstoffatomen bedeutet, sowie ihre Salze mit anorganischen oder organischen Säuren.

6. Verbindungen nach Anspruch 5, dadurch gekennzeichnet, daß D geradkettiges oder verzweigtes Alkyl oder Alkoxy mit 1 bis 5 Kohlenstoffatomen ist.

7. 3-Methylthiomethyl-7-[[2-(2-aminothiazol-4-yl)-2-(2-bromethyl)-oximino[-acetamido]-ceph-3-em-4-carbonsäure-syn-isomer und 7-[[2-(aminothiazol-4-yl)2-[(carboxymethoxyimino]-acetyl]-amino-3-(methylthiomethyl)-ceph-3-em-4-carbonsäure-syn-isomer sowie ihre Salze mit Alkalimetallen, Erdalkalimetallen, Magnesium, Ammoniak, organischen Aminobasen, Säuren und ihre leicht spaltbaren Ester.

8. Verfahren zur Herstellung der Verbindung der Formel (I') nach Anspruch 1, dadurch gekennzeichnet, daß eine Verbindung der Formel (II),

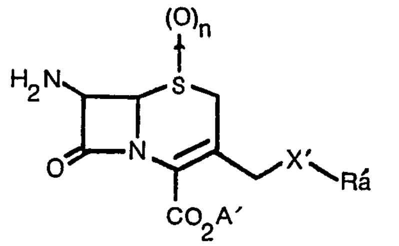

(II)

in der n, X' und R'a die in Anspruch 1 angegebene Bedeutung haben und A' Wasserstoff oder einen leicht abspaltbaren Esterrest bedeutet, mit einer Verbindung der Formel (III) oder einem ihrer funktionellen Derivate,

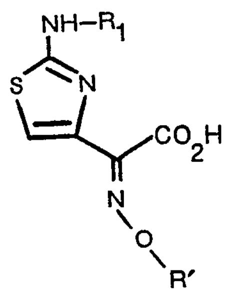

(III)

in der bedeuten: $R_1$ Wasserstoff oder eine Aminoschutzgruppe,

R' entweder geradkettiges oder verzweigtes Alkyl, Alkenyl, Alkinyl oder Cycloalkyl mit höchstens 6 Kohlenstoffatomen, oder Acetyl, Propionyl, Butylryl, Valeryl, Hexanoyl, Acryloyl, Crotonoyl, Benzoyl oder Carbamoyl, wobei jeder dieser Reste mit einem oder mehreren Resten, ausgewählt unter Carboxyl, gegebenenfalls als Salz oder Ester, Alkoxycarbonyl, Carbamoyl, Dimethylcarbamoyl, Amino, Alkylamino, Dialkylamino, Halogen, Alkoxy, Alkylthio, Aryl, heterozyklisches Aryl, Arylthio, heterozyklisches Arylthio, gegebenenfalls mit Alkyl substituiert, substituiert ist oder Alkoxycarbonyl, zu einer Verbindung der Formel (IV) umgesetzt wird,

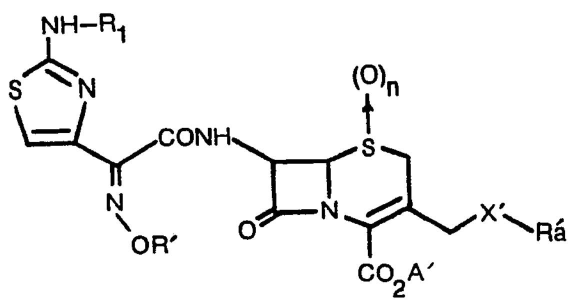

(IV)

in der $R_1$, R', A', R'a, X' und n die oben angegebene Bedeutung haben, diese Verbindung gegebenenfalls, wenn n 0 ist und X' Schwefel oder Sauerstoff bedeutet, mit einem Oxidationsmittel zu einer Verbindung der Formel (IV) oxidiert wird, in der n 1 oder 2 ist und X' oxidierten Schwefel in Form von Sulfoxyd oder Sulfon oder Sauerstoff bedeutet, und gegebenenfalls, wenn notwendig oder erwünscht, einer oder mehreren der folgenden Reaktionen in beliebig er Reihenfolge unterworfen wird:

a) Abspalten durch Hydrolyse, Hydrierung oder Einwirkung von Thioharnstoff aller oder eines Teils der Estergruppen oder der Schutzgruppen der Aminogruppe (n),

b) Veresterung oder Salzbildung mit einer Base der Carboxylgruppe (n),

c) Salzbildung mit einer Säure der Aminogruppe (n).

9. Verfahren zur Herstellung der Verbindungen der Formel (I') nach Anspruch 1, dadurch gekennzeichnet, daß eine Verbindung der Formel (V),

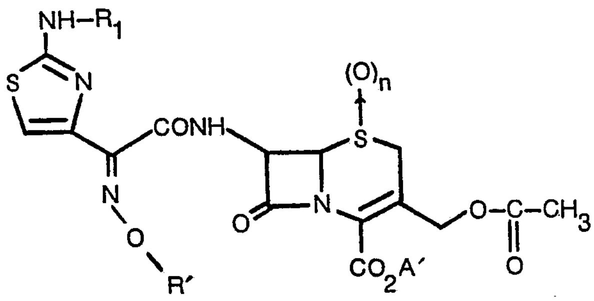

(V)

in der $R_1$, R', A' und n die in Anspruch 8 angegebene Bedeutung haben, mit einer Verbindung der Formel R'a—SH in der R'a die in Anspruch 1 angegebene Bedeutung hat, oder zuerst mit 2-Mercaptopyridin-N-oxyd und dann mit einer Verbindung der Formel R'aOH, in der R'a die oben angegebene Bedeutung hat, zu einer Verbindung der Formel (IV), wie oben definiert, umgesetzt wird, die Verbindung der Formel (IV) gegebenenfalls, wenn n 0 ist und X' Schwefel oder Sauerstoff bedeutet, mit einem Oxidationsmittel zu einer Verbindung der Formel (IV) oxidiert wird, in der n 1 oder 2 ist und X' oxidierten Schwefel in Form eines Sulfoxyds oder Sulfons oder Sauerstoff bedeutet, und gegebenenfalls, wenn notwendig oder erwünscht, einer oder mehreren der folgenden Reaktionen in beliebiger Reihenfolge unterworfen wird:

a) Abspalten durch Hydrolyse, Hydrierung oder Einwirkung von Thioharnstoff aller oder eines Teils der Estergruppen oder der Aminoschutzgruppen,

b) Veresterung oder Salzbildung mit einer Base der Carboxylgruppe (n)

c) Salzbildung mit einer Säure der Aminogruppe (n).

10. Verfahren nach Anspruch 8 zur Herstellung der Verbindungen der Formel (I'), dadurch gekennzeichnet, daß zur Durchführung des Verfahrens eine Verbindung der Formel (III), in der $R_1$ eine Aminoschutzgruppe ist, und als funktionelles Derivat der Säure der Formel (III) ein gemischtes Carbonsäure-Sulfonsäure-anhydrid verwendet wird.

11. Verfahren zur Herstellung der Verbindungen der Formel (I') nach Anspruch 1, dadurch gekennzeichnet, daß eine Verbinduing (II'),

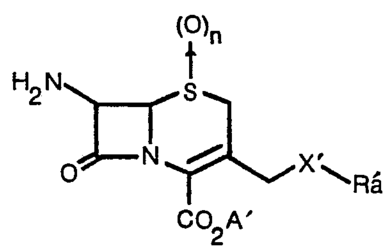

(II')

in der R'a, n, X' und A' die in Anspruch 1 angegebene Bedeutung haben, mit einer Verbindung der Formel (III') oder einem ihrer funktionellen Derivate,

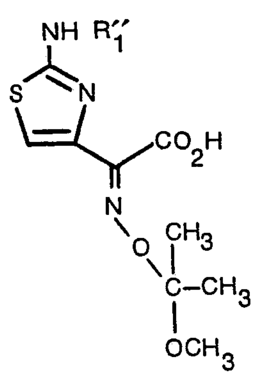

(III')

in der $R''_1$ eine Aminoschutzgruppe ist, zu einer Verbindung (IV') umgesetzt wird,

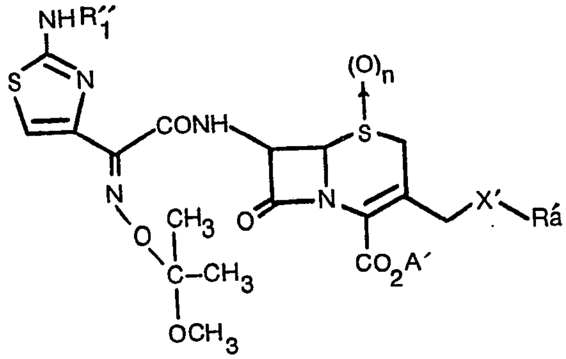

(IV′)

in der A′, R″$_1$, R′a, X′ und n die obenangegebene Bedeutung haben, die Verbindung der Formel (IV′) mit einer Säure unter milden Bedingungen zu einer Verbindung der Formel (VI) umgesetzt wird,

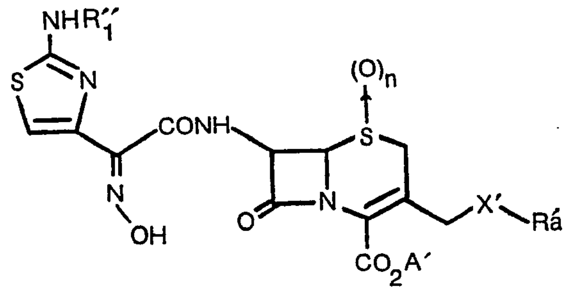

(VI)

aus dieser Verbindung gegebenenfalls ein Ester oder Salz gebildet wird und die Verbindung in Gegenwart einer Base mit einem funktionellen Derivat der Formel

Rd-Hal

in der bedeuten: Rd geradkettiges oder verzweigtes Alkyl, Alkenyl, Alkinyl oder Cycloalkyl mit höchstens 6 Kohlenstoffatomen, oder Acetyl, Propionyl, Butyryl, Valeryl, Hexanoyl, Acryloyl, Crotonoyl, Benzoyl oder Carbamoyl, wobei jeder dieser Reste gegebenenfalls mit einem oder mehreren Resten substituiert ist, ausgewählt unter Carboxyl, gegebenenfalls als Salz oder Ester, Alkoxycarbonyl, Carbamoyl, Dimethylcarbamoyl, Amino, Alkylamino, Dialkylamino, Halogen, Alkoxy, Alkylthio, Aryl, heterozyklisches Aryl, Arylthio, gegebenenfalls mit einem Alkyl substituiertes heterozylisches Arylthio, oder Alkoxycarbonyl und Hal Halogen, zu einer Verbindung der Formel (VII) umgesetzt wird,

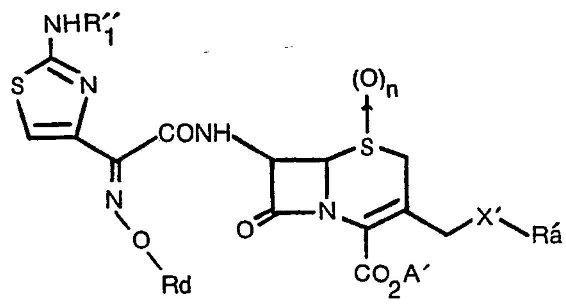

(VII)

in der R″$_1$, R′a, Rd, A′, X′ und n die obenangegebene Bedeutung haben, die Verbindung der Formel (VII) hydrolysiert, hydriert oder mit Thioharnstoff zur Entfernung der Aminoschutzgruppe R″$_1$ umgesetzt wird und, wenn gewünscht oder notwendig, einer oder mehreren der folgenden Reaktionen in beliebiger Reihenfolge unterworfen wird:

a) Abspalten der Estergruppe (n),
b) Verestern oder Salzbildung mit einer Base der Carboxylgruppe (n),
c) Salzbildung mit einer Säure der Aminogruppe (n).

12. Verbindungen der Formel (I′) nach Anspruch 1 sowie ihre pharmazeutisch verträglichen Säuresalze als Medikament.

13. Verbindungen der Formel (I) nach einem der Ansprüche 2 bis 4 sowie ihre pharmazeutisch verträglichen Säuresalze als Medikament.

14. Verbindungen nach Anspruch 5 oder 6 sowie ihre pharmazeutisch verträglichen Säuresalze als Medikament.

15. Verbindungen nach Anspruch 7 sowie ihre pharmazeutisch verträglichen Säuresalze als Medikament.

16. Pharmazeutische Zusammensetzungen mit einem Gehalt an mindestens einem Medikament nach einem der Ansprüche 12 bis 15 als Wirkstoff.

**Patentansprüche für den Vertragsstaat: AT**

1. Verfahren zur Herstellung von Verbindungen der allgemeinen Formel (I')

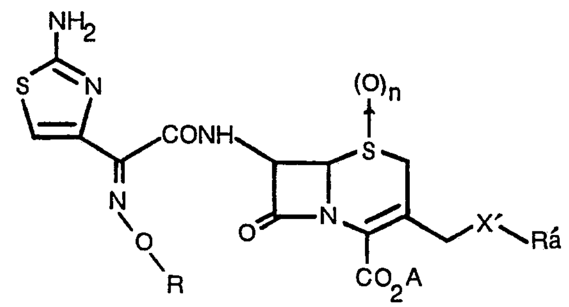

(I')

Syn-Isomere in der R, A, X', R'a und n die folgenden Bedeutungen haben oder

A] R bedeutet entweder geradkettiges oder verzweigtes Alkyl, Alkenyl, Alkinyl oder Cycloalkyl mit höchstens 6 Kohlenstoffatomen, oder Acetyl, Propionyl, Butyryl, Valeryl, Hexanoyl, Acryloyl, Crotonoyl, Benzoyl, Carbamoyl, wobei jeder dieser Reste gegebenenfalls substituiert ist mit einem oder mehreren Resten, ausgewählt unter Carboxyl, gegebenenfalls als Salz oder Ester, Methoxycarbonyl, Ethoxycarbonyl, Carbamoyl, Dimethylcarbamoyl, Amino, Methylamino, Dimethylamino, Halogen, Methoxy, Ethoxy, Propyloxy, Methylthio, Ethylthiophenyl, Tetrazolyl, Phenylthio, Tetrazolylthio oder Thiadiazolylthio, gegebenenfalls mit Methyl substituiert, oder Methoxycarbonyl oder Ethoxycarbonyl

A bedeutet: Wasserstoff, Alkalimetall, Erdalkalimetall, Magnesium, Ammonium oder eine organische Aminobase oder einen leicht abspaltbaren Esterrest,

R'a bedeutet: geradkettiges oder verzweigtes Alkyl, gegebenenfalls unterbrochen durch ein Heteroatom, Alkenyl oder Alkinyl mit höchstens 6 Kohlenstoffatomen, Benzyl oder Phenylethyl, gegebenenfalls substituiert mit Carboxyl, Amino oder Dimethylaminoethyl,

n 0, 1 oder 2 ist,

X' gegebenenfalls oxidierten Schwefel in Form von Sulfoxyd oder Sulfon oder Sauerstoff bedeutet,

wobei R nicht Alkenyl oder Alkinyl mit höchsten 6 Kohlenstoffatomen oder geradkettiges oder verzweigtes Alkyl mit höchstens 6 Kohlenstoffatomen, gegebenenfalls substituiert mit einem oder mehreren Resten, ausgewählt unter Carboxyl, gegebenenfalls als Salz oder Ester, Alkoxycarbonyl, Amino, Alkylamino, Dialkylamino heterozyklisches Aryl, bedeutet, wenn der Rest —X'—R'a Alkoxy, Alkylthio oder Alkenylthio ist, oder

B] n 0 ist,

A Wasserstoff bedeutet und

R entweder $—CH_2—CH_2—NH_2$, $—CH_2CO_2H$ oder $—C(CH_3)_2\ CO_2H$ bedeutet,

Z' Sauerstoff oder Schwefel und

R'a Methyl oder Ethyl ist, oder

$$\underset{\displaystyle CH_3}{R—\underset{|}{C}H—CO_2H} \text{ ist,}$$

X' Sauerstoff oder Schwefel und

R'a Methyl bedeutet, sowie die Salze der Verbindungen der Formel (I') mit anorganischen oder organischen Säuren, dadurch gekennzeichnet, daß eine Verbindung der Formel (II)

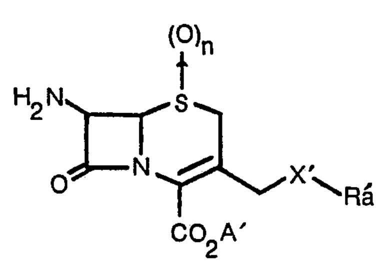

(II)

in der n, X' und R'a die oben agegebene Bedeutung haben und A' Wasserstoff oder einen leicht abspaltbaren Esterrest bedeutet, mit einer Verbindung der Formel (III) oder einem ihrer funktionellen Derivate,

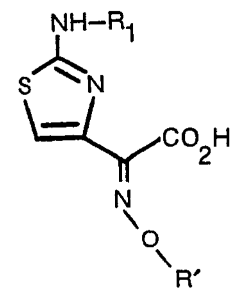

(III)

in der bedeuten: $R_1$ Wasserstoff oder eine Aminoschutzgruppe,

R' entweder geradkettiges oder verzweigtes Alkyl, Alkenyl, Alkinyl oder Cycloalkyl mit höchstens 6 Kohlenstoffatomen, oder Acetyl, Propionyl, Butylryl, Valeryl, Hexanoyl, Acryloyl, Crotonoyl, Benzoyl oder Carbamoyl, wobei jeder dieser Reste mit einem oder mehreren Resten, ausgewählt unter Carboxyl, gegebenenfalls als Salz oder Ester, Alkoxycarbonyl, Carbamoyl, Dimethylcarbamoyl, Amino, Alkylamino, Dialkylamino, Halogen, Alkoxy, Alkylthio, Aryl, heterozyklisches Aryl, Arylthio, heterozyklisches Arylthio, gegebenenfalls mit Alkyl substituiert, substituiert ist oder Alkoxycarbonyl, zu einer Verbindung der Formel (IV) umgesetzt wird,

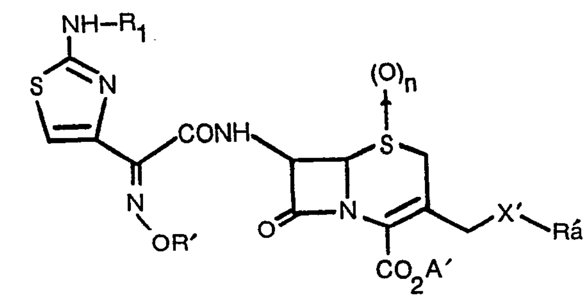

(IV)

in der $R_1$, R', A', R'a, X' und n die oben angegebene Bedeutung haben, diese Verbindung gegebenenfalls, wenn n 0 ist und X' Schwefel oder Sauerstoff bedeutet, mit einem Oxidationsmittel zu einer Verbindung der Formel (IV) oxidiert wird, in der n 1 oder 2 ist und X' oxidierten Schwefel in Form von Sulfoxyd oder Sulfon oder Sauerstoff bedeutet, und gegebenenfalls, wenn notwendig oder erwünscht, einer oder mehreren der folgenden Reaktionen in beliebiger Reihenfolge unterworfen wird:

a) Abspalten durch Hydrolyse, Hydrierung oder Einwirkung von Thioharnstoff aller oder eines Teils der Estergruppen oder der Schutzgruppen der Aminogruppe (n),

b) Veresterung oder Salzbildung mit einer Base der Carboxylgruppe (n),

c) Salzbildung mit einer Säure der Aminogruppe (n).

2. Verfahren zur Herstellung der Verbindungen der Formel (I') nach Anspruch 1, dadurch gekennzeichnet, daß eine Verbindung der Formel (V),

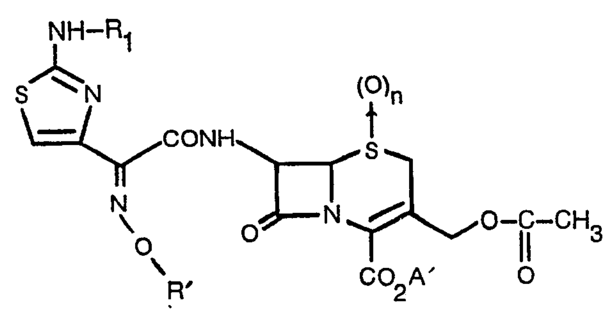

(V)

in der $R_1$, R', A' und n die in Anspruch 1 angegebene Bedeutung haben, mit einer Verbindung der Formel R'a—SH in der R'a die in Anspruch 1 agegebene Bedeutung hat, oder zuerst mit 2-Mercaptopyridin-N-oxyd und dann mit einer Verbindung der Formel R'aOH, in der R'a die oben angegebene Bedeutung hat, zu einer Verbindung der Formel (IV), wie oben definiert, umgesetzt wird, die Verbindung der Formel (IV) gegebenenfalls, wenn n 0 ist und X' Schwefel oder Sauerstoff bedeutet, mit einem Oxidationsmittel zu einer Verbindung der Formel (IV) oxidiert wird, in der n 1 oder 2 ist und X' oxidierten Schwefel in Form eines Sulfoxyds oder Sulfons oder Sauerstoff bedeutet, und gegebenenfalls, wenn notwendig oder

erwünscht, einer oder mehreren der folgenden Reaktionen in beliebiger Reihenfolge unterworfen wird:

a) Abspalten durch Hydrolyse, Hydrierung oder Einwirkung von Thioharnstoff aller oder eines Teils der Estergruppen oder der Aminoschutzgruppen,

b) Veresterung oder Salzbildung mit einer Base der Carboxylgruppe (n)

c) Salzbildung mit einer Säure der Aminogruppe (n).

3. Verfahren nach Anspruch 1 zur Herstellung von Verbindungen der Formel (I'), dadurch gekennzeichnet, daß zur Durchführung des Verfahrens eine Verbindung der Formel (III), in der $R_1$ eine Aminoschutzgruppe ist, und als funktionelles Derivat der Säure der Formel (III) ein gemischtes Carbonsäure-Sulfonsäure-anhydrid verwendet wird.

4. Verfahren nach einem der Ansprüche 1 bis 3 zur Herstellung der Verbindungen nach Anspruch 1 der allgemeinen Formel (I)

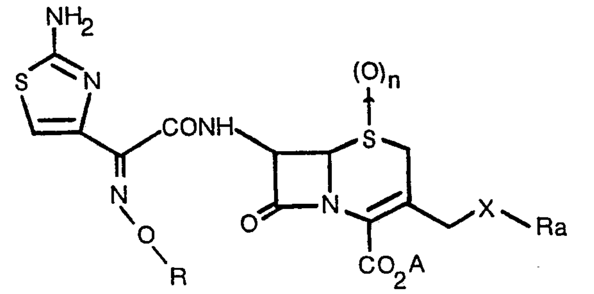

(I)

Syn-Isomere

in der R, A und n wie in Anspruch 1 definiert sind, Ra ein geradkettiges oder verzweigtes Alkyl, Alkenyl oder Alkinyl mit höchstens 6 Kohlenstoffatomen oder gegebenenfalls substituiertes Arylalkyl bedeutet und

X Schwefel oder Sauerstoff ist, sowie der Salze der Verbindungen der Formel (I) mit anorganischen oder organischen Säuren, dadurch gekennzeichnet, daß am Anfang Verbindungen der Formeln (II), R'aSH und R'aOH verwendet werden, in denen X' und R'a die oben für X und Ra angegebene Bedeutung haben.

5. Verfahren zur Herstellung von Verbindungen der Formel (I') nach Anspruch 1, dadurch gekennzeichnet, daß eine Verbindung (II'),

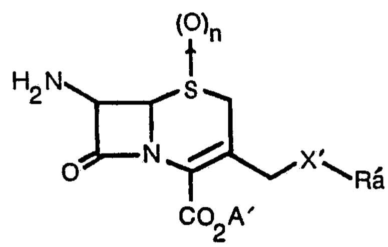

(II')

in der R'a, n, X' und A' die in Anspruch 1 angegebene Bedeutung haben, mit einer Verbindung der Formel (III') oder einem ihrer funktionellen Derivate,

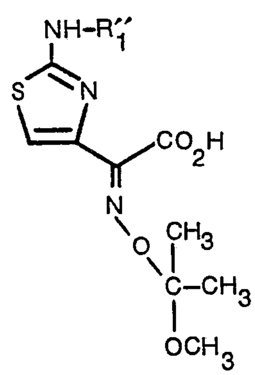

(III')

in der R''$_1$ eine Aminoschutzgruppe ist, zu einer Verbindung (IV') umgesetzt wird,

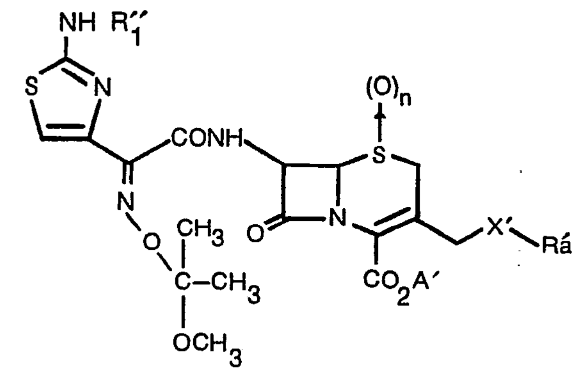

(IV')

in der A', R''$_1$, R'a, X' und n die obenangegebene Bedeutung haben, die Verbindung der Formel (IV') mit einer Säure unter milden Bedingungen zu einer Verbindung der Formel (VI) umgesetzt wird,

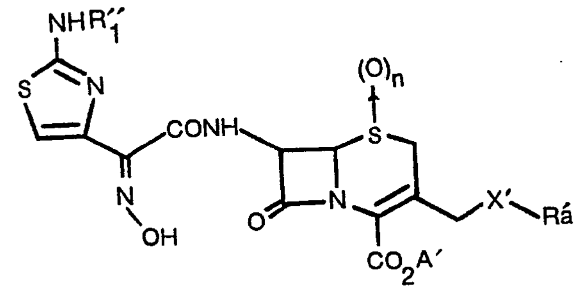

(VI)

aus dieser Verbindung gegebenenfalls ein Ester oder Salz gebildet wird und die Verbindung in Gegenwart einer Base mit einem funktionellen Derivat der Formel

Rd-Hal

in der bedeuten: Rd geradkettiges oder verzweigtes Alkyl, Alkenyl, Alkinyl oder Cycloalkyl mit höchstens 6 Kohlenstoffatomen, oder Acetyl, Propionyl, Butyryl, Valeryl, Hexanoyl, Acryloyl, Crotonoyl, Benzoyl, Carbamoyl oder Alkoxycarbamoyl, wobei jeder dieser Reste gegebenenfalls mit einem oder mehreren Resten substituiert ist, und Hal Halogen, zu einer Verbindung der Formel (VII) umgesetzt wird,

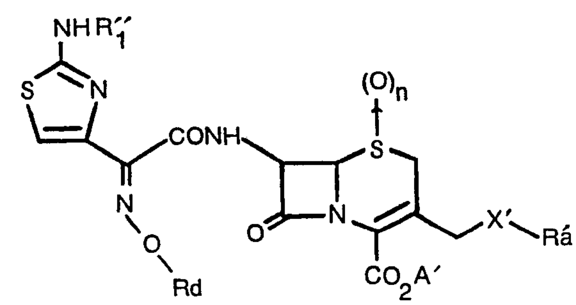

(VII)

in der R''$_1$, R'a, Rd, A', X' und n die obenangegebene Bedeutung haben, die Verbindung der Formel (VII) hydrolysiert, hydriert oder mit Thioharnstoff zur Entfernung der Aminoschutzgruppe R''$_1$ umgesetzt wird und, wenn gewünscht oder notwendig, einer oder mehreren der folgenden Reaktionen in beliebiger Reihenfolge unterworfen wird:

a) Abspalten der Estergruppe (n),

b) Verestern oder Salzbildung mit einer Base der Carboxylgruppe (n),

c) Salzbildung mit einer Säure der Aminogruppe (n).

6. Verfahren nach Anspruch 4, dadurch gekennzeichnet, daß eine Verbindung der Formel (III), in der R' geradkettiges oder verzweigtes Alkyl mit 1 bis 4 Kohlenstoffatomen, das gegebenenfalls mit einer freien oder veresterten Carboxylgruppe oder mit einer Aminogruppe substituiert ist, und eine Verbindung der Formel (II) oder R'aSH oder R'aOH verwendet werden, in denen R'a Alkyl mit höchstens 6 Kohlenstoffatomen ist.

7. Verfahren nach Anspruch 5, dadurch gekennzeichnet, daß R'a Methyl und in der Verbindung der Formel (II) n 0 ist.

8. Verfahren nach Anspruch 4, dadurch gekennzeichnet, daß die eventuelle Endveresterung mit einem funktionellen Derivat des Restes der Formel

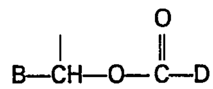

durchgeführt wird, in der B Wasserstoff oder geradkettiges oder verzweigtes Alkyl mit 1 bis 5 Kohlenstoffatomen und D geradkettiges oder verzweigtes Alkyl oder Alkoxy mit 1 bis 15 Kohlenstoffatomen ist.

9. Verfahren nach Anspruch 8, dadurch gekennzeichnet, daß D geradkettiges oder verzweigtes Alkyl oder Alkoxy mit 1 bis 5 Kohlenstoffatomen ist.

10. Verfahren zur Herstellung von Verbindungen der Formel (I'), wie nach einem Ansprüche 6 bis 9 erhaltenen Verbindungen, dadurch gekennzeichnet, daß gemäß dem Verfahren gemäß Anspruch 5 vorgegangen wird.

11. Verfahren nach Anspruch 4 oder 10, dadurch gekennzeichnet, daß die folgende Verbindung der Formel (I) hergestellt wird:
3-Methylthiomethyl-7-[[2-(2-aminothiazol-4-yl)-2-(2-bromethyl)-oximino[-acetamido]-ceph-3-em-4-carbonsäure-syn-isomer sowie ihre Salze mit Alkalimetallen, Erdalkalimetallen, Magnesium, Ammoniak, organischen Aminobasen, Säuren und leicht spaltbaren Ester.

**Claims for Contracting States: BE CH DE GB IT LI LU NL SE**

1. Products of general formula (I'):

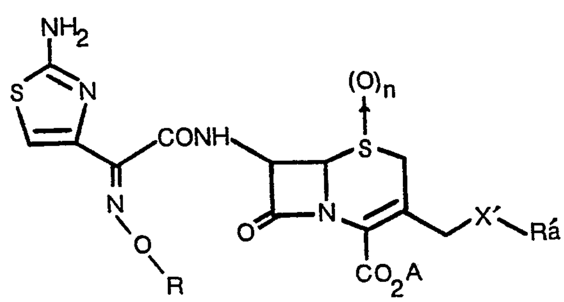

(I')

*syn* isomers in which R, A, X', R'a, n have the following significances either A] R represents,

*either* a linear or branched alkyl, alkenyl, alkynyl or cycloalkyl radical having at most 6 carbon atoms,

*or* an acetyl, propionyl, butyryl, valeryl, hexanoyl, acryloyl, crotonyl, benzoyl or carbamoyl radical, each of these radicals being optionally substituted by one or more radicals chosen from the following radicals; carboxy optionally salified or esterified, methoxy, carboxyl, ethoxy carbonyl, carbamoyl, amino dimethylcarbamoyl, methylamino, dimethylamino, halogen, methoxy, ethoxy, propyloxy, methylthio, phenyl ethylthio, tetrazolyl, phenylthio, tetrazolylthio or thiadiazolylthio optionally substituted by methyl,

*or* R represents a methoxycarbonyl or ethoxycarbonyl radical.

A represents a hydrogen atom, an equivalent of an alkali metal, of an alkaline earth metal, of magnesium, of ammonium or of an aminated organic base, or A represents the remainder of an easily cleavable ester group,

R'a represents an alkyl radical optionally interrupted by a heteroatom, linear or branched alkenyl or alkynyl radical having at most 6 carbon atoms, or a benzyl or phenylethyl radical optionally substituted by a carboxy, amino or dimethylaminoethyl radical.

n is equal to 0, 1 or 2.

X' represents a sulphur atom optionally oxidised in the form of the sulphoxide or sulphone, or an oxygen atom, it being understood that R does not represent an alkenyl or alkynyl radical having at most 6 carbon atoms or a linear or branched alkyl radical having at most 6 carbon atoms optionally substituted by one or more radicals chosen from the following, carboxy optionally salified or esterified, alkoxycarbonyl, amino, alkylamino, dialkylamino or heterocyclic aryl, when the radical —X'—R'a represents an alkoxy, alkylthio or alkenylthio radical;

or B] n is equal to 0, A represents a hydrogen atom and *either* R represents a $—CH_2—CH_2—NH_2$, $—CH_2CO_2H$ or $—C(CH_3)_2CO_2H$ radical, X' represents a sulphur or oxygen atom and R'a represents a methyl

or ethyl radical

*or* R represents a

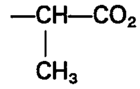

radical, X' represents an oxygen or sulphur atom and R'a represents a methyl radical as well as the salts of products of formula (I') with mineral or organic acids.

2. Products according to claim 1, corresponding to general formula (I)

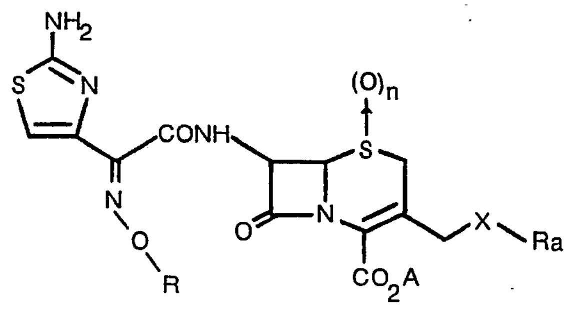

(I)

*syn* isomer

in which R, A and n are as previously defined in claim 1, Ra represents a linear or branched alkyl radical, alkenyl or alkynyl radical having at most 6 carbon atoms, or a benzyl or phenylethyl radical optionally substituted by a carboxy, amino or dimethyl aminoethyl radical and X represents a sulphur or oxygen atom, as well as the salts of products of formula (I) with mineral or organic acids.

3. Products of general formula (I) as defined in claim 2, in which R represents a linear or branched alkyl radical having from 1 to 4 carbon atoms, substituted by an esterified or salified free carboxylic radical, or by an amino radical and Ra represents an alkyl radical having at most 6 carbon atoms.

4. Products of general formula (I) as defined in claims 2 or 3, in which Ra represents a methyl radical and n represents the number 0.

5. Products according to claim 2, corresponding to the general formula ($I_A$):

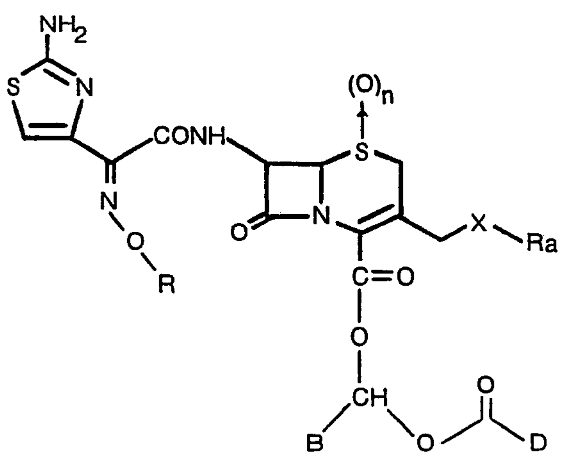

($I_A$)

in which R, n, X and Ra are as previously defined in claim 2, B represents a hydrogen atom or a linear or branched alkyl radical containing from 1 to 5 carbon atoms and D represents a linear or branched alkyl or alkoxy radical containing from 1 to 15 carbon atoms as well as their salts with mineral or organic acids.

6. Products according to claim 5, characterised in that D represents a linear or branched alkyl or alkoxy radical containing from 1 to 5 carbon atoms.

7. 3 - methylthiomethyl - 7 - [[2 - (2 - amino - thiazol - 4 - yl) - 2 - (2 - bromoethyl) - oximino] - acetamido] - ceph - 3 - eme - 4 - carboxylic acid, *syn* isomer and 7 - [[2 - (aminothiazol - 4 - yl) - 2[ - (carboxy - methoxy - imino]acetyl] - amino - 3 - (methylthiomethyl) - ceph - 3 - eme - 4 - carboxylic acid, *syn* isomer, as well as their salts with alkali metals, alkaline-earth metals, magnesium, ammonium hydroxide, aminated organic bases, acids and their easily cleavable esters.

8. Preparation process of products of general formula (I') as defined in claim 1, characterised in that a product of formula (II):

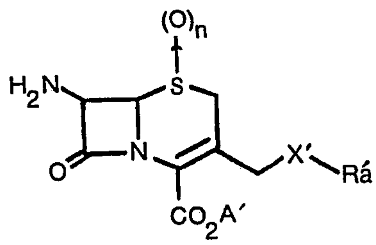

(II)

in which n, X' and R'a have the significance indicated in claim 1 and A' represents a hydrogen atom or the remainder of an easily cleavable ester group, is treated by a product of formula (III)

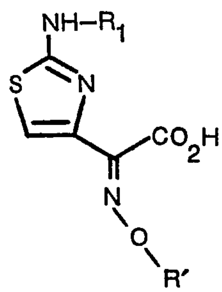

(III)

or a functional derivative of this acid, formula (III) in which $R_1$ represents a hydrogen atom or a protector group of the amino radical and either R' represents a linear or branched alkyl radical, an alkenyl, alkynyl or cycloalkyl radical having at most 6 carbon atoms, or R' represents one of the following radicals, acetyl, propionyl, butyryl, valeryl, hexanoyl, acryloyl, crotonoyl, benzoyl, carbamoyl, each of these radicals being optionally substituted by one or more radicals chosen from the following, carboxy optionally salified or esterified, alkoxycarbonyl, carbamoyl, dimethylcarbamoyl, amino, alkylamino, dialkylamino, halogen, alkoxy, alkylthio, aryl, heterocyclic aryl, arylthio, heterocyclic arylthio optionally substituted by alkyl,

or R' represents an alkoxycarbonyl radical to obtain a product of formula (IV):

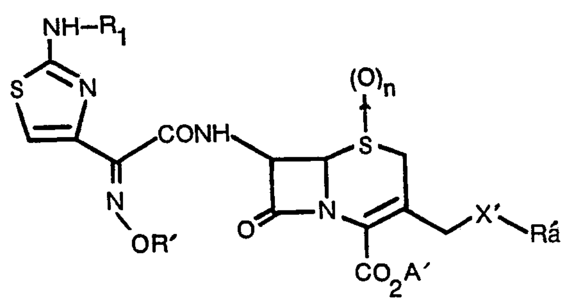

(IV)

in which $R_1$, R', A' R'a, X' and n have the previous significance, which product, if desired, in the case where n is equal to 0 and X' represents a sulphur or oxygen atom, is treated with an oxidising agent, to obtain a product of formula (IV) in which n is equal to 1 or 2 and X' represents an oxidised sulphur atom in the form of the sulphoxide or sulphone or an oxygen atom and, which product of formula IV, if necessary or desired, is subjected to one or more of the following reactions, in any order:

a) detachment by hydrolysis, hydrogenolysis or by the action of thiourea of all or part of the ester groups or the protector groups of the amino radical or radicals.

b) esterification or salification of the carboxylic radical or radicals by a base.

c) salification of the amino radical or radicals by an acid.

9. Preparation process of products of formula (I') as defined in claim 1, characterised in that a product of formula (V):

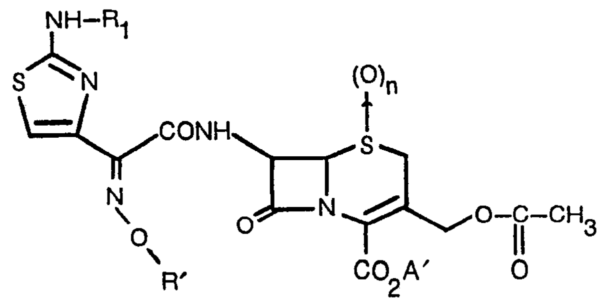

(V)

in which $R_1$, R', A' and n have the significance indicated in claim 8, is treated *either* by a product of formula R'a-SH in which R'a has the significance indicated in claim 1, or *or* firstly by 2-mercapto-pyridine-N-oxide then by a product of formula R'aOH in which R'a has the previous significance, to obtain a product of formula IV, as defined previously, which product of formula IV, if desired, in the case where n is equal to 0 and X' represents a sulphur or oxygen atom, is treated with an oxidising agent, to obtain a product of formula (IV) in which n is equal to 1 or 2 and X' represents an oxidised sulphur atom in the form of the sulphoxide or sulphone or an oxygen atom and, product of formula IV which if necessary or desired is subjected to one or more of the following reactions, in any order:

a) detachment by hydrolysis, hydrogenolysis or by the action of thiourea of all or part of the ester groups or protector groups of the amino radical or radicals.

b) esterification or salification of the carboxylic radical or radicals by a base.

c) salification of the amino radical or radicals by an acid.

10. Preparation process according to claim 8 of products of formula (I'), characterised in that a product of formula (III) in which $R_1$ represents a protector group of the amino radical, is used to implement the process and in that the functional derivative of the acid of formula (III) is a mixed carboxylic sulphonic anhydride.

11. Preparation process of products of formula (I'), as defined in claim 1, characterised in that a product of formula (II'):

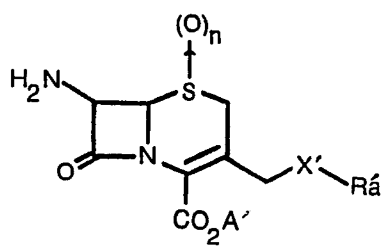

(II')

in which R'a, n, X' and A' have the significance indicated in claim 1, is treated by a product of formula (III')

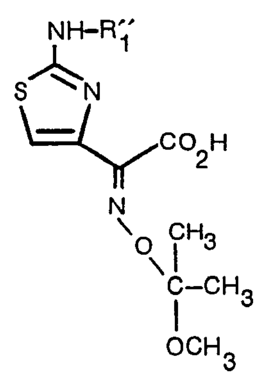

(III')

or a functional derivative of this acid, formula (III') in which $R''_1$ represents a protector group of the amino radical, to obtain a product of formula (IV'):

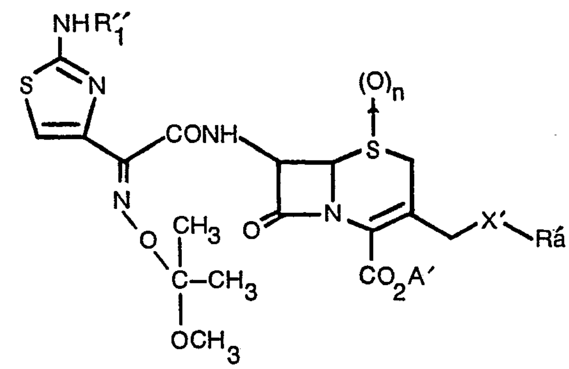

(IV')

in which A', $R''_1$, R'a, X' and n have the previous significance, which product of formula (IV') is treated by an acid in moderate conditions, to obtain a product of formula VI:

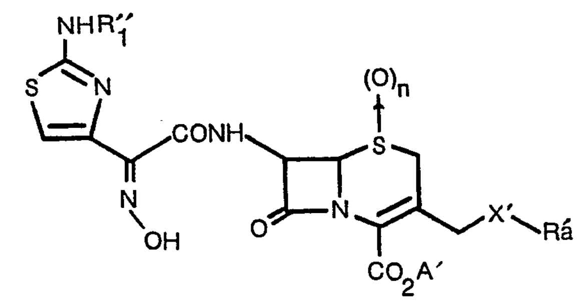

(VI)

which product, if desired, is esterified or salified and treated in the presence of a base by a functional derivative of formula:

Rd-Hal

in which Rd represents *either* a linear or branched alkyl radical, an alkenyl, alkynyl or cycloalkyl radical having at most 6 carbon atoms,

*or* an acetyl, propionyl, butyryl, valeryl, hexanoyl, acryloyl, crotonoyl, benzoyl or carbamoyl radical each of these radicals being optionally substituted by one or more radicals chosen from the following, carboxy optionally salified or esterified, alkoxycarbonyl, carbamoyl, dimethylcarbamoyl, amino, alkylamino, dialkylamino, halogen, alkoxy, alkylthio, aryl, heterocyclic aryl, arylthio, heterocyclic arylthio optionally substituted by alkyl,

*or* Rd represents an alkoxycarbonyl radical and Hal represents a halogen atom, to obtain a product of formula VII:

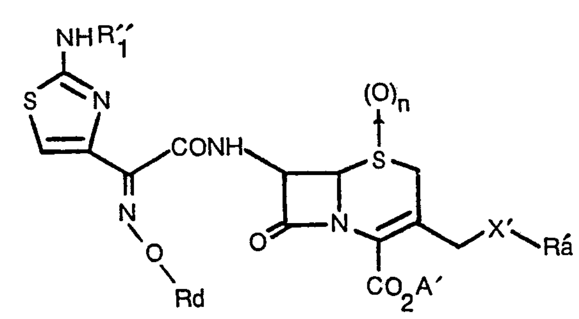

(VII)

in which $R''_1$, R'a, Rd, A', X' and n have the significance indicated above and which product of formula VII is subjected to hydrolysis, hydrogenolysis or to the action of thiourea to eliminate the protector radical $R''_1$ of the amino radical and, if desired or if necessary, to one or more of the following reactions, in any order:

a) elimination of the ester group or groups,

b) esterification or salification of the amino radical or radicals by a base,

c) salification of the amino radical or radicals by an acid.

12. As medicaments, the products corresponding to formula (I') as defined in claim 1, as well as their pharmaceutically acceptable acid salts.

13. As medicaments, the products of formula (I) as defined in any one of claims 2, 3, and 4 as well as their pharmaceutically acceptable acid salts.

14. As medicaments, the products as defined in claim 5 or 6, as well as their pharmaceutically acceptable acid salts.

15. As medicaments, the products as defined in claim 7, as well as their pharmaceutically acceptable salts.

16. Pharmaceutical compositions containing, as active principle, at least one of the medicaments according to one of claims 12 to 15.

**Claims for Contracting State: AT**

1. Preparation process for products of general formula (I')

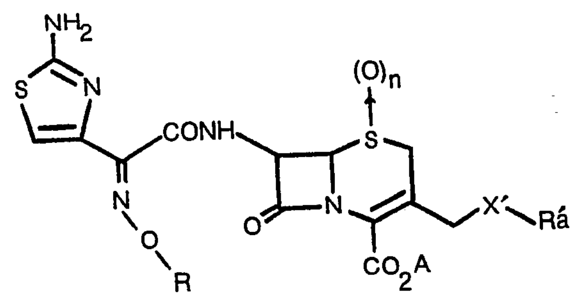

(I')

*syn* isomers in which R, A, X', R'a, n have the following significances *either* A] R represents,

*either* a linear or branched alkyl radical, an alkenyl, alkynyl or cycloalkyl radical having at most 6 carbon atoms,

carbamoyl each of these radicals being optionally substituted by one or more of the following radicals, carboxy optionally salified or esterified, methoxy carboxyl, ethoxy carbonyl, carbamoyl, amino-dimethylcarbamoyl, methylamino, dimethylamino, halogen, methoxy, ethoxy, propyloxy, methylthio, phenyl ethylthio, tetrazolyl, phenylthio, tetrazolylthio or thiadiazolylthio optionally substituted by methyl,

*or* R represents a methoxycarbonyl or ethoxycarbonyl radical.

A represents a hydrogen atom, an equivalent of an alkali metal, of an alkaline earth metal, of magnesium, of ammonium or of an aminated organic base, or A represents the remainder of an easily cleavable ester group,

R'a represents an alkyl radical optionally interrupted by a heteroatom, linear or branched alkenyl or alkynyl radical having at most 6 carbon atoms, or a benzyl or phenylethyl radical optionally substituted by a carboxy, amino or dimethylaminoethyl radical.

n is equal to 0, 1 or 2.

X' represents a sulphur atom optionally oxidised in the form of the sulphoxide or sulphone, or an oxygen atom, it being understood that R does not represent an alkenyl or alkynyl radical having at most 6 carbon atoms or a linear or branched alkyl radical having at most 6 carbon atoms optionally substituted by one or more of the following carboxy optionally salified or esterified, alkoxycarbonyl, amino, alkylamino, dialkylamino or heterocyclic aryl, when the —X'—R'a radical represents an alkoxy, alkylthio or alkenylthio radical;

*or* B] n is equal to 0, A represents a hydrogen atom and *either* R represents a $CH_2—CH_2—NH_2$, $—CH_2CO_2H$ or $—C(CH_3)_2CO_2H$ radical, X' represents a sulphur or oxygen atom and R'a represents a methyl or ethyl radical

*or* R represents a

$$\underset{\displaystyle CH_3}{\overset{}{CH}}—CO_2H$$

radical, X' represents an oxygen or sulphur atom and R'a represents a methyl radical as well as the salts of products of formula (I') with mineral or organic acids characterised in that a product of formula (II)

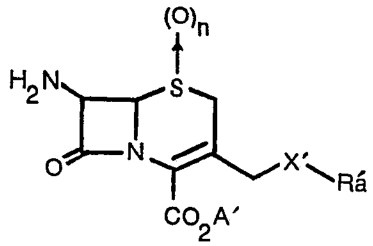

(II)

in which n, X' and R'a have the significance indicated previously and A' represents a hydrogen atom or the remainder of an easily cleavable ester group, is treated by a product of formula (III):

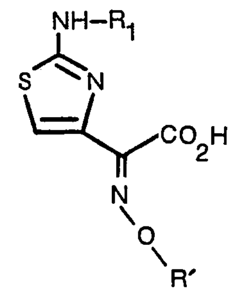

(III)

or a functional derivative of this acid, formula (III) in which $R_1$ represents a hydrogen atom or a protector group of the amino radical and either R' represents a linear or branched alkyl radical, an alkenyl, alkynyl or cycloalkyl radical having at most 6 carbon atoms, or R' represents one of the following radicals, acetyl, propionyl, butyryl, valeryl, hexanoyl, acryloyl, crotonoyl, benzoyl, carbamoyl or alkoxycarbonyl, each of these radicals being optionally substituted, to obtain a product of formula (IV):

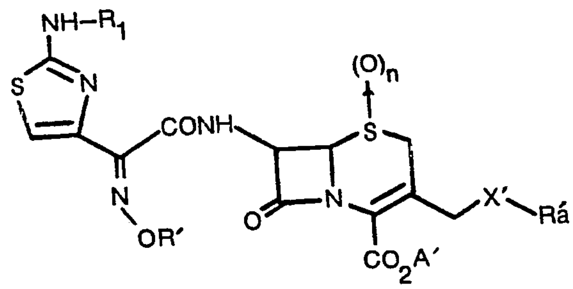

(IV)

in which $R_1$, R', A' R'a, X' and n have the previous significance, which product, if desired, in the case where n is equal to 0 and X' represents a sulphur or oxygen atom, is treated with an oxidation agent, to obtain a product of formula (IV) in which n is equal to 1 or 2 and X' represents a sulphur atom oxidised in the form of the sulphoxide or sulphone or an oxygen atom and, which product of formula IV, if necessary or if desired, is subjected to one or more of the following reactions, in any order:

a) detachment by hydrolysis, hydrogenolysis or by the action of thiourea of all or part of the ester groups or the protector groups of the amino radical or radicals.

b) esterification or salification of the carboxylic radical or radicals by a base.

c) salification of the amino radical or radicals by an acid.

2. Preparation process of products of formula (I') as defined in claim 1, characterised in that a product of formula (V):

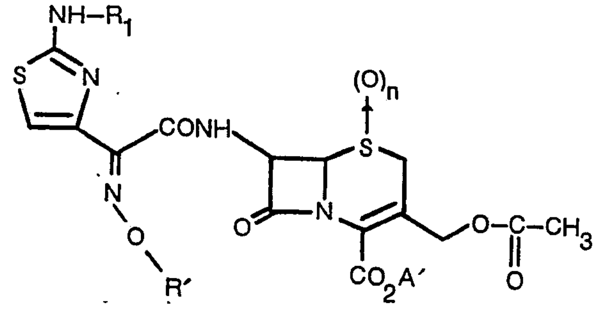

(V)

in which $R_1$, R', A' and n have the significance indicated in claim 1, is treated *either* by a product of formula R'a-SH in which R'a has the significance indicated in claim 1, *or* firstly by 2-mercapto-pyridine-N-oxide then by a product of formula R'aOH in which R'a has the previous significance, to obtain a product of formula IV, as defined previously, which product of formula IV, if desired, in the case where n is equal to 0 and X' represents a sulphur or oxygen atom, is treated with an oxidising agent, to obtain a product of formula (IV) in which n is equal to 1 or 2 and X' represents an oxidised sulphur atom in the form of the sulphoxide or sulphone or an oxygen atom and which product of formula IV is, if necessary or desired, subjected to one or more of the following reactions, in any order:

a) detachment by hydrolysis, hydrogenolysis or by the action of thiourea of all or part of the ester groups or protector groups of the amino radical or radicals.

b) esterification or salification of the carboxylic radical or radicals by a base.

c) salification of the amino radical or radicals by an acid.

3. Preparation process according to claim 1 of products of formula (I'), characterised in that for the implementation of the process, a product of formula (III) is used, in which $R_1$ represents a protector group of the amino radical and in that the functional derivative of the acid of formula (III) is a mixed carboxylic sulphonic anhydride.

4. Process according to any one of claims 1, 2 and 3, for the preparation of products according to claim 1, corresponding to the general formula (I):

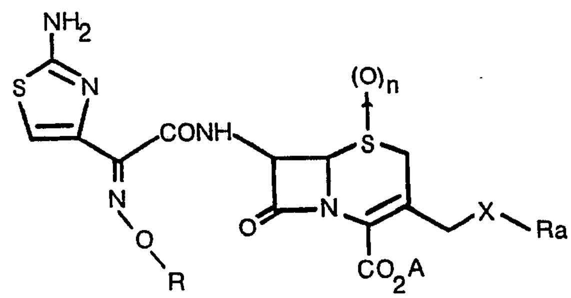

(I)

*syn* isomers

in which R, A and n are as defined in claim 1, Ra represents a linear or branched alkyl, alkenyl or alkynyl radical having at most 6 carbon atoms, or an optionally substituted aralkyl radical, and X represents a sulphur or oxygen atom, as well as the salts of products of formula (I) with mineral or organic acids, characterised in that at the outset products of formula (II), R'aSH and R'aOH are used, in which X' and R'a have the values indicated previously for X and Ra.

5. Preparation process of products of formula (I'), as defined in claim 1, characterised in that a product of formula (II'):

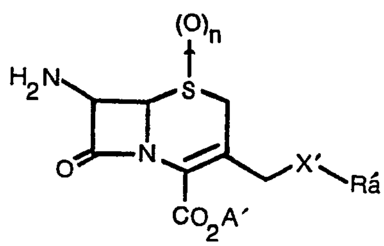

(II')

in which R'a, n, X' and A' have the significance indicated in claim 1, is treated by a product of formula (III'):

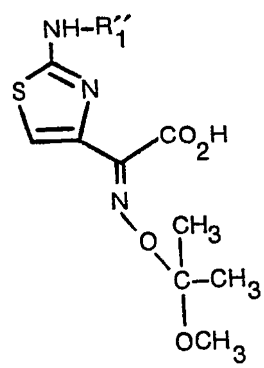

(III')

or a functional derivative of this acid, formula (III') in which $R''_1$ represents a protector group of the amino radical, to obtain a product of formula (IV'):

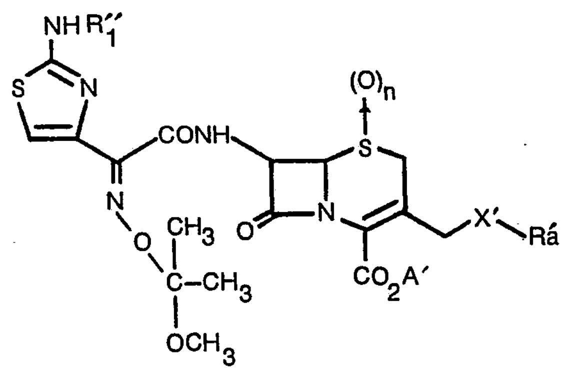

(IV')

in which A', $R''_1$, R'a, X' and n have the previous significance, product of formula (IV') which is treated by an acid in moderate conditions, to obtain a product of formula VI:

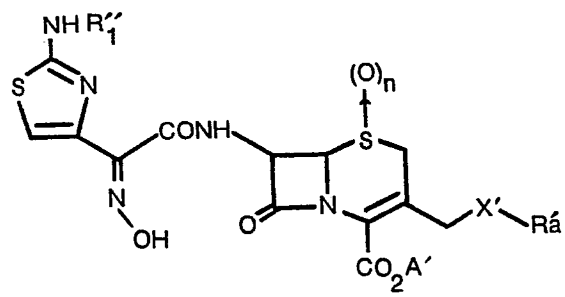

(VI)

a product which, if desired, is esterified or salified and treated in the presence of a base by a functional derivative of formula:

Rd-Hal

in which Rd represents a linear or branched alkyl radical, an alkenyl, alkynyl or cycloalkyl radical having at most 6 carbon atoms, an acetyl, propionyl, butyryl, valeryl, hexanoyl, acryloyl, crotonoyl, benzoyl, carbamoyl or alkoxycarbonyl radical, each of these radicals being optionally substituted and Hal represents a halogen atom, to obtain a product of formula VII:

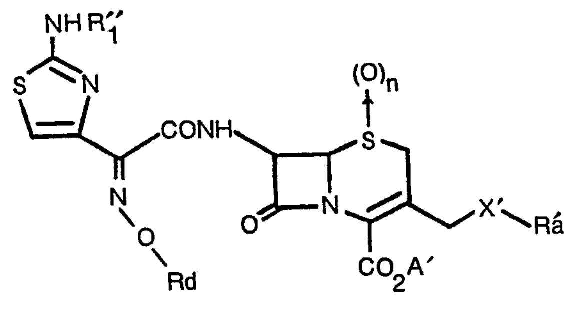

(VII)

in which $R''_1$, R'a, Rd, A', X' and n have the significance indicated above and which product of formula VII is subjected to hydrolysis, hydrogenolysis or to the action of thiourea to eliminate the $R''_1$ protector radical of the amino radical and, if desired or necessary, to one or more of the following reactions, in any order:

a) elimination of the ester group or groups,

b) esterification or salification of the carboxylic radical or radicals by a base,

c) salification of the amino radical or radicals by an acid.

6. Process according to claim 4, characterised in that in the product of formula (III), R' represents a linear or branched alkyl radical having from 1 to 4 carbon atoms, optionally substituted by a free or esterified carboxylic radical or by an amino radical and in that the product of formula (II) or in the product of

formula R'aSH or R'aOH, R'a represents an alkyl radical having at most 6 carbon atoms.

7. Process according to claim 5, characterised in that R'a represents a methyl radical and in that in the product of formula (II), n is equal to 0.

8. Process according to claim 4, characterised in that the optional final esterification is effected by a functional derivative of a group of formula

$$B-CH-\overset{\overset{O}{|}}{O}-C-D,$$

in which B represents a hydrogen atom or a linear or branched alkyl radical containing from 1 to 5 carbon atoms and D represents a linear or branched alkyl or alkoxy radical containing from 1 to 15 carbon atoms.

9. Process according to claim 7, characterised in that D represents a linear or branched alkyl or alkoxy radical containing from 1 to 5 carbon atoms.

10. Preparation process of products of formula (I'), as obtained in any one of claims 6 to 9, characterised in that the operation is carried out according to the process of claim 5.

11. Process according to claim 4 or 10, characterised in that the product corresponding to the following formula (I) is prepared:

- 3 - methylthiomethyl - 7 - [[2 - (2 - amino - thiazol - 4 - yl) - 2 - (2 - bromoethyl) - oxyimino] - acetamido] - ceph - 3 - eme - 4 - carboxylic acid *syn* isomer, as well as its salts with alkali metals, alkaline-earth metals, magnesium, amonium hydroxide, aminated organic bases, acids and its easily cleavable esters.